(19) **Europäisches Patentamt**
**European Patent Office**
**Office européen des brevets**

(11) **EP 4 625 316 A1**

(12) **EUROPEAN PATENT APPLICATION**

(43) Date of publication:
**01.10.2025 Bulletin 2025/40**

(21) Application number: **24465514.8**

(22) Date of filing: **26.03.2024**

(51) International Patent Classification (IPC):
**G06T 7/00** (2017.01)

(52) Cooperative Patent Classification (CPC):
**G06T 7/0012;** A61B 6/032; G06N 3/045;
G06T 2207/10081; G06T 2207/20081;
G06T 2207/20084; G06T 2207/30056;
G06T 2207/30096

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **CHABIN, Guillaume**
**75012 Paris (FR)**
• **XU, Zhoubing**
**Princeton, NJ, 08540 (US)**

• **TANABENE, Asma**
**92340 Bourg-la-Reine (FR)**
• **DRAGHIA, Alin Madalin**
**500256 Brasov (RO)**
• **SCHWIER, Michael**
**22529 Hamburg (DE)**
• **WANG, Jianing**
**Plainsboro, NJ, 08536 (US)**

(74) Representative: **Kraus & Lederer PartGmbB**
**Thomas-Wimmer-Ring 15**
**80539 München (DE)**

Remarks:
Amended claims in accordance with Rule 137(2)
EPC.

(54) **A SYSTEM AND A METHOD FOR THREE-DIMENSIONAL LESION SEGMENTATION USING MULTI-PHASE COMPUTED TOMOGRAPHY OR MAGNETIC RESONANCE IMAGING DATA**

(57) Computer-implemented methods and computer systems are provided for locating a liver lesion in the input data. In an example, a computer implemented method of the current disclosure comprises receiving, by a first computer interface, input data; applying a trained function to the input data; generating, by the trained function, output data, wherein the output data is associated with the input data; providing, via a second computer interface, the output data, wherein the input data comprises a multi-phase three-dimensional, 3D, computed tomography, CT, imaging dataset of a liver lesion associated with a patient, and wherein the output data comprises a three-dimensional, 3D, segmentation data for locating the liver lesion in the input data.

FIG 5

**Description**

Independent of the grammatical term usage, individuals with male, female or other gender identities are included within the term.

Technical field

Various examples of the disclosure generally pertain to segmentation of imaging datasets. Various examples specifically pertain to generating three-dimensional lesion segmentation data using multi-phase computed tomography or multi-phase magnetic resonance imaging data associated with the lesion.

Background

The segmentation of organs and other regions of interest plays a crucial role in diagnostic imaging. The purpose of such segmentation is manifold. It can either directly be used for visualization purposes, such as typically conducted for liver examination, or as a surrogate or intermediate result for algorithms analyzing said organ.

In clinical practice, both texture and shape of segmented regions are important factors for the assessment in diverse fields, such as oncology.

More particularly, in a clinical setting where a three-dimensional (3D) computed tomography (CT) is used, radiologists often examine several CT phases to select one as the reference phase where a lesion such as liver lesion is most distinctly visible. Features are then calculated specifically on this phase for subsequent analysis. Additionally, the radiologists typically use multi-phase information to study enhancement patterns and compare the lesion's appearance across multiple phases, contributing to more accurate lesion delineation.

To outline the lesion, radiologists may use either manual methods or semi-automatic software. For semi-automatic segmentation, the software typically takes an initial diameter of a target lesion marked by a radiologist. Then the software typically generates a 3D outline of the target lesion, and then provides a re-estimated diameter based on the predicted outline.

NPL1: "Ronneberger, O., Fischer, P., & Brox, T. (2015). U-net: Convolutional networks for biomedical image segmentation; Medical Image Computing and Computer-Assisted Intervention-MICCAI 2015: 18th International Conference, Munich, Germany, October 5-9, 2015, Proceedings, Part III 18 (pp. 234-241), Springer International Publishing" describes a neuronal network architecture "U-Net" for automatic segmentation of liver lesions. U-Net can be trained on either all-phase data or specific phase data to produce either phase-independent or phase-specific networks.

NPL2: "Ouhmich, Farid, et al., Liver tissue segmentation in multiphase CT scans using cascaded convolutional neural networks; International journal of computer assisted radiology and surgery 14 (2019): 1275-1284" describes two deep learning approaches to handle multi-phase CT scans. The first approach concatenates single-phase images at the input layer. The second approach independently generates output maps for each phase and subsequently fuses them to derive the final segmentation.

NPL3: "Xu, Yingying, et al., PA-ResSeg: A phase attention residual network for liver tumor segmentation from multiphase CT images; Medical Physics 48.7 (2021): 3752-3766" describes applying an attention mechanism to capture cross-phases information in the context of 2D CT scans.

NPL4: "Zhang, Yue, et al., Multi-phase liver tumor segmentation with spatial aggregation and uncertain region inpainting; Medical Image Computing and Computer Assisted Intervention-MICCAI 2021: 24th International Conference, Strasbourg, France, September 27-October 1, 2021, Proceedings, Part I 24, Springer International Publishing, 2021" describes multi-phase segmentation using spatial aggregation in 2D context.

The prior art describes machine learning methods that primarily rely on single-phase imaging through phase-agnostic or phase-specific neural networks (e.g., NPL1 or NPL2). Some machine learning methods employ neural network architectures leveraging multi-phase information, but they are confined to two-dimensional, 2D, applications (e.g., NPL4). Alternatively, some methods deploy 3D models that are trained separately on single-phase data and then attempt to combine the results from these phase-specific models during post-processing in order to generate 3D output data (e.g., NPL3).

Thus, there is a need to provide an improved computer implemented method for enhanced 3D segmentation of lesions.

Summary

There is a need for a computer implemented method and a system for generating enhanced 3D segmentation data of a lesion or another object of interest directly from CT or MRI multi-phase imaging datasets of the lesion.

This need is met by the features of the independent claims. The features of the dependent claims define embodiments.

Hereinafter, techniques of determining segmentation data will be disclosed. The segmentation data corresponds to location information for locating an object of interest. The segmentation data may include a segmentation map: the segmentation map identifies for each pixel or voxel of imaging data whether the respective pixel or voxel as part or not part of the segmented object. Other forms of segmentation data include splines or surfaces outlining the circumference of the segmented object. Yet another form of segmentation data includes a heat map. A heat map can include a probabilistic measure of a certain pixel or voxel of the imaging data being part or not part of the segmented object. Still another option for segmentation data includes bounding boxes or bounding spheres.

In a first aspect, a computer-implemented method for providing output data is provided. The computer-implemented method comprises receiving, by a first computer interface, input data. The computer-implemented method further comprises applying a trained function to the input data. The computer-implemented method further comprises generating, by the trained function, output data, wherein the output data is associated with the input data. The computer-implemented method further comprises providing, via a second computer interface, the output data. The input data comprises a multi-phase three-dimensional, 3D, computed tomography, CT, imaging dataset of a liver lesion associated with a patient, and the output data comprises a three-dimensional, 3D, segmentation data for locating the liver lesion in the input data.

In an example, the trained function for the computer-implemented method of the first aspect may comprise an encoder part for encoding each 2D slice of each phase P1, P2, ..., Pn from the multi-phase 3D CT imaging dataset into feature information F1, F2, F3. The trained function for the computer-implemented method of the first aspect may further comprise a spatial aggregation, SA, module for aggregating the encoded feature information F1, F2, F3 into spatially aggregated feature information Agg_F4. The trained function for the computer-implemented method of the first aspect may further comprise a decoder part for decoding the spatially aggregated feature information Agg_F4 into the 3D segmentation data. The encoder part may comprise a first branch receiving 2D slices of the input data associated with a reference phase P1 and a second branch receiving 2D slices of the input data associated with one or more remaining phases P2, ..., Pn, each of the first branch and the second branch providing the encoded feature information F1, F2, F3 to the SA module for aggregation. Each of the first branch and the second branch of the encoder part may comprise multiple stacked layers LE1, ..., Lem of a neuronal network providing the encoded feature information F1, F2, F3 to the SA module for aggregation into the spatially aggregated feature information Agg_F4.

In an example, the input data for the computer-implemented method may be pre-processed by a pre-processing algorithm. The pre-processing algorithm may comprise manually selecting, for each phase from the multi-phase 3D CT imaging dataset, a 2D slice having a specific characteristic. An example would be that 2D slice in which a target lesion has the largest diameter. The pre-processing algorithm may further comprise indicating a center or a diameter of the target lesion and receiving, via the first computer interface, the center or the diameter of the target lesion as a part of the input data. Alternatively to manually selecting, for each phase from the multi-phase 3D CT imaging dataset, the 2D slice, the pre-processing algorithm may comprise automatically detecting, by an additional trained function, a bounding-box or coordinates of a centroid associated with the target lesion on a 2D slice for each phase from the multi-phase 3D CT imaging dataset wherein the target lesion has the largest diameter, and receiving, via the first computer interface, by the trained function, the bounding-box or the coordinates of the centroid as a part of the input data. The additional trained function may comprise a deep neuronal network or a convolutional neuronal network configured to receive the input data and provide the bounding box or the coordinates of the centroid to the trained function as a part of the input data.

In an example, the pre-processing algorithm may further comprise registering 2D slices of the multi-phase 3D CT imaging dataset associated with different phases P1, ..., Pn according to a mesh-based algorithm, or according to a point-cloud-based algorithm. The mesh-based algorithm may comprise providing a mesh having a plurality of interconnected data nodes representing a geometrical structure of a lesion within images of the multi-phase 3D CT imaging dataset for aligning or matching 2D slices associated with each phase P1, ..., Pn from the multi-phase 3D CT imaging dataset to the geometrical structure of the lesion. The point-cloud-based algorithm may comprise providing a point cloud comprising a plurality of data points representing the geometrical structure of the lesion within the images of the multi-phase 3D CT imaging dataset for identifying a geometrical transformation that aligns corresponding points in the point cloud for spatial correspondence between the 2D slices of every phase P1, ..., Pn from the multi-phase 3D CT imaging dataset.

In an example, the output data for the computer-implemented method of the first aspect may be post-processed by a post-processing algorithm. The post-processing algorithm may comprise rendering, by a computer processor, the 3D segmentation data. The post-processing algorithm may further comprise calculating, by the computer processor, one or more parameters associated with the target lesion from the 3D segmentation data. For instance, the one or more parameters may comprise any one of a largest diameter, a volume, a lesion type, a lesion's contour, a minimum and/or a maximum intensity of each lesion. The post-processing algorithm may further comprise providing the one or more parameters via the second computer interface. The 3D segmentation data may comprise a three dimensional, 3D, heat map and/or three dimensional segmentation mask obtainable by generating, by the trained function, the three-dimensional, 3D, heatmap and converting the generated 3D heatmap into a 3D binary mask by applying a predefined threshold to the generated 3D heatmap and identifying one or more connected components within the binary mask.

In a second aspect, a computer system is provided. The computer system comprises a first interface configured for receiving input data. The computer system further comprises a second interface configured for providing output data. The computer system further comprises a computation unit. The computation unit comprises a computer processor configured for applying a trained function trained by a machine learning algorithm to the input data. The output data according to the second aspect is generated by the trained function. The input data according to the second aspect comprises a multi-phase three-dimensional, 3D, computed tomography, CT, imaging dataset of a liver lesion associated with a patient. The output data according to the second aspect comprises a three-dimensional, 3D, segmentation data for locating the liver lesion in the input data.

In an example, the trained function for the computer system of the second aspect may comprise an encoder part for encoding each 2D slice of each phase P1, P2, ..., Pn from the multi-phase 3D CT imaging dataset into feature information F1, F2, F3. The trained function may further comprise a spatial aggregation, SA, module for aggregating the encoded feature information F1, F2, F3 into spatially aggregated feature information Agg_F4. The trained function may further comprise a decoder part for decoding the spatially aggregated feature information Agg_F4 into the 3D segmentation data. The encoder part may comprise a first branch receiving 2D slices of the input data associated with a reference phase P1 and a second branch receiving 2D slices of the input data associated with one or more remaining phases P2, ..., Pn, each of the first branch and the second branch providing the encoded feature information F1, F2, F3 to the SA module for aggregation. Each of the first branch and the second branch of the encoder part may comprise multiple stacked layers LE1, ..., Lem of a neuronal network providing the encoded feature information F1, F2, F3 to the SA module for aggregation into the spatially aggregated feature information Agg_F4.

In an example, the computation unit of the computer system of the second aspect may be further configured to pre-process the input data according to a pre-processing algorithm. The pre-processing algorithm may comprise manually selecting, for each phase from the multi-phase 3D CT imaging dataset, a 2D slice, wherein a target lesion has the largest diameter. The pre-processing algorithm may further comprise indicating a center or a diameter of the target lesion and receiving, via the first computer interface, the center or the diameter of the target lesion as a part of the input data. Alternatively to manually selecting, for each phase from the multi-phase 3D CT imaging dataset, the 2D slice, the pre-processing algorithm may comprise automatically detecting, by an additional trained function, location information such as a bounding-box or coordinates of a centroid associated with the target lesion on a 2D slice for each phase from the multi-phase 3D CT imaging dataset wherein the target lesion has the largest diameter, and receiving, via the first computer interface, by the trained function, the bounding-box or the coordinates of the centroid as a part of the input data. The additional trained function may comprise a deep neuronal network such as a convolutional neuronal network configured to receive the input data and provide the bounding box or the coordinates of the centroid to the trained function as a part of the input data.

In an example, the pre-processing algorithm may further comprise registering 2D slices of the multi-phase 3D CT imaging dataset associated with different phases P1, ..., Pn according to a mesh-based algorithm, or according to a point-cloud-based algorithm. The mesh-based algorithm may comprise providing a mesh having a plurality of interconnected data nodes representing a geometrical structure of a lesion within images of the multi-phase 3D CT imaging dataset for aligning or matching 2D slices associated with each phase P1, ..., Pn from the multi-phase 3D CT imaging dataset to the geometrical structure of the lesion. The point-cloud-based algorithm may comprise providing a point cloud comprising a plurality of data points representing the geometrical structure of the lesion within the images of the multi-phase 3D CT imaging dataset for identifying a geometrical transformation that aligns corresponding points in the point cloud for spatial correspondence between the 2D slices of every phase P1, ..., Pn from the multi-phase 3D CT imaging dataset.

In an example, the computation unit of the computer system of the second aspect may be further configured to post-process the output data according to a post-processing algorithm. The post-processing algorithm may comprise rendering, by a computer processor, the 3D segmentation data.

The post-processing algorithm may further comprise calculating, by the computer processor, one or more parameters associated with the target lesion from the 3D segmentation data, wherein the one or more parameters may comprise any one of a largest diameter, a volume, a lesion type, a lesion's contour, a minimum and/or a maximum intensity of each lesion. The post-processing algorithm may comprise providing the one or more parameters via the second computer interface. The 3D segmentation data may comprise a three-dimensional, 3D, heat map and/or three dimensional segmentation mask obtainable by generating, by the trained function, the three-dimensional, 3D, heatmap and converting the generated 3D heatmap into a 3D binary mask by applying a predefined threshold to the generated 3D heatmap and identifying one or more connected components within the binary mask.

In a third aspect, a computer implemented method for providing a trained function is provided. The computer implemented method of the third aspect comprises receiving, by a first training computer interface, training input data. The computer implemented method of the third aspect further comprises providing, by a computer processor, the training input data to a training function. The computer implemented method of the third aspect further comprises embedding, by the training function, the training input data. The computer implemented method of the third aspect further comprises receiving, via a second training computer interface, output training data, wherein the training output data is associated with the training input data. The computer implemented method of the third aspect further comprises generating, by the computer processor, a trained function comprising embedded training input data. The computer implemented method of the third aspect further comprises providing, via a third training computer interface, the trained function for use. The training input data for the computer implemented method of the third aspect comprises one or more multi-phase three-dimensional, 3D, computed tomography, CT, imaging datasets of one or more liver lesions, wherein each imaging dataset is associated with a patient.

In an example, the trained function, provided by the computer-implemented method of the third aspect, may comprise an encoder part for encoding each 2D slice of each phase P1, P2, ..., Pn from the imaging datasets into feature information F1, F2, F3. The trained function, provided by the computer-implemented method of the third aspect, may further comprise a spatial aggregation, SA, module for aggregating the encoded feature information F1, F2, F3 into spatially aggregated feature information Agg_F4. The trained function, provided by the computer-implemented method of the third aspect, may further comprise a decoder part for decoding the spatially aggregated feature information Agg_F4 into the 3D segmentation data. The encoder part may comprise a first branch receiving 2D slices of the imaging datasets associated with a reference phase P1 and a second branch receiving 2D slices of the imaging datasets associated with one or more remaining phases P2, ..., Pn, each of the first branch and the second branch providing the encoded feature information F1, F2, F3 to the SA module for aggregation. Each of the first branch and the second branch of the encoder part may comprise multiple stacked layers LE1, ..., Lem of a neuronal network providing the encoded feature information F1, F2, F3 to the SA module for aggregation into the spatially aggregated feature information Agg_F4.

In an example, the computer-implemented method of the third aspect may further comprise detecting one or more regions of interest, ROIs, for each lesion within each image of the multi-phase 3D CT imaging dataset. The detecting may comprise determining a centre of mass or centroid of each lesion. The computer-implemented method of the third aspect may further comprise cropping each image of the multi-phase 3D CT imaging dataset around the centre of mass or centroid. The computer-implemented method of the third aspect may further comprise encoding cropped images by the encoder part. The computer-implemented method of the third aspect may further comprise matching the centres of mass or centroids across the the multi-phase 3D CT imaging dataset to establish a representation of the lesion's position in three-dimensional, 3D, space. The computer-implemented method of the third aspect may further comprise aggregating, by the SA module, encoded feature information F1, F2, F3 into spatially aggregated feature information Agg_F4. The computer-implemented method of the third aspect may further comprise generating, by the decoder part decoding the spatially aggregated feature information Agg_F4, a three-dimensional, 3D, heat map, wherein the 3D heat map may be a 3D graphical representation indicating spatial location of each lesion across the multi-phase 3D CT imaging dataset. The computer-implemented method of the third aspect may further comprise generating, by applying a threshold to the generated 3D heatmap, a 3D segmentation mask.

In an example, the computer-implemented method of the third aspect may further comprise training an additional function comprising a deep neuronal network to provide an additional trained function, the additional function being trained by embedding additional training data associated with a plurality of bounding-boxes or coordinates of a plurality of centroids each associated with a lesion present in the training input data provided to the additional function together with the plurality of bounding-boxes or coordinates of the plurality of centroids via the first training computer interface. The additional trained function may be trained to generate output data being a bounding-box or coordinates of a centroid associated with a target lesion. The computer-implemented method of the third aspect may further comprise chaining the additional trained function with the trained function of the first and/or the second aspects such that the output data generated by the additional trained function may be provided as an additional input data in addition to the input data provided to the trained function for automatic detection of a bounding-box or coordinates of a centroid associated with a target lesion present in the input data.

In an example, the computer-implemented method of the third aspect may further comprise receiving, by the first training computer interface, a three-dimensional, 3D, ground truth segmentation mask corresponding to the 3D heatmap. The computer-implemented method of the third aspect may further comprise calculating a weighted loss function by integrating a cross-entropy loss component and a dice loss component, wherein the cross-entropy loss component may quantify dissimilarity between predicted and actual probability distributions of class labels, wherein the actual probability distribution may be a binary vector representing the true class, where the probability is one for the correct class representing a lesion and zero for other classes that do not represent a lesion. The dice loss component may quantify the spatial overlap between predicted and the ground truth segmentation masks. The computer-implemented method of the third aspect may further comprise iteratively updating one or more hyperparameters of the trained function based on the calculated weighted loss function, wherein the one or more hyperparameters may comprise any one of or a combination thereof a type of optimizer, a learning rate and/or a regularization weight. The computer-implemented method of the third aspect may further comprise generating the trained function comprising the one or more hyperparameters.

In an example, the computer-implemented method of the third aspect may further comprise evaluating the trained function according to an evaluation algorithm. The evaluation algorithm may comprise receiving, via the first training computer interface, the training input data by the trained function, wherein the trained function is to be evaluated by the evaluation algorithm for use in the method of the first and/or second aspect. The evaluation algorithm may further comprise generating, by the trained function, a 3D heatmap. The evaluation algorithm may comprise generating a binary mask from the generated 3D heatmap by applying a predetermined threshold to the 3D heatmap. The evaluation algorithm may further comprise identifying a 3D segmentation mask that aligns with the center of the lesion based on identifying connected components within the binary mask by: identifying spatial proximity between pixels of the binary mask, assigning one or more labels to the one or more connected components for distinguishing the one or more components, generating an output representation comprising the labelled connected components. The evaluation algorithm may further comprise generating the identified 3D segmentation mask and providing the generated 3D segmentation mask via the second training computer interface as output training data. The evaluation algorithm may further comprise providing, via the first training computer interface, a comparative ground truth segmentation mask associated with the training input data. The evaluation algorithm may further comprise comparing the generated 3D segmentation mask with the comparative ground truth segmentation mask, wherein the comparative ground truth segmentation mask may be an annotated set of binary images that may serve as a reference delineating the true boundaries or labels of the connected components. The evaluation algorithm may further comprise calculating an evaluation metric by comparing the generated 3D segmentation mask and the comparative ground truth segmentation mask, the evaluation metric comprising one or more performance characteristics of the trained function. The evaluation algorithm may further comprise providing the calculated evaluation metric via the second or the third training computer interface.

According to a fourth aspect, a computer program product is provided. The computer program product comprises computer readable instructions which, when executed by a computer processor, cause the computer processor to carry out the method of the first and/or the third aspects.

According to a fifth aspect, a training system is provided. The training system comprises a first training computer interface configured for receiving training input data. The training system further comprises a training computation unit. The training computation unit comprises a computer processor configured for training a training function by providing the training input data to the training function for embedding, by the training function, the training input data. The computer processor is further configured for generating the trained function comprising the embedded training data. The training system further comprises a second training computer interface configured for providing output data, wherein the output data is associated with the input data. The training system further comprises a third training computer interface configured for providing the trained function for use. The training input data comprises one or more multi-phase three-dimensional, 3D, computed tomography, CT, imaging datasets of one or more liver lesions, wherein each imaging dataset is associated with a patient.

The training function of the third and/or fifth aspects may be used for the computer-implemented method of the first aspect and/or the computer-implemented method of the third aspect, and vice versa.

It is to be understood that the features mentioned above and those yet to be explained below may be used not only in the respective combinations indicated, but also in other combinations or in isolation without departing from the scope of the invention.

Brief description of figures

Fig. 1 illustrates a flow chart for using a trained function for three-dimensional segmentation of a lesion;

Fig. 2 illustrates a system for using the trained function according to fig. 1;

Fig. 3 illustrates a flow chart for training a training function for use according to fig. 1;

Fig. 4 illustrates a system for training the training function of fig. 3;

Fig. 5 illustrates an encoder-decoder architecture with an integrated spatial aggregator module for three-dimensional segmentation of figs. 1-4;

Fig. 6 illustrates a deep neuronal network for the encoder-decoder neural network architecture of fig. 5; and

Fig. 7 illustrates a convolutional neuronal network for the encoder-decoder neural network architecture of fig. 5.

Detailed description

In the following the solution according to the invention is described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the system.

Furthermore, in the following the solution according to the invention is described with respect to methods and systems for use as well as with respect to methods and systems for training of a function (a machine learning algorithm, ML comprising, e.g., a neuronal network). Features, advantages or alternative embodiments herein can be assigned to the other claimed objects and vice versa. In other words, claims for methods and systems for training can be improved with features described or claimed in context of the methods and systems for use, and vice versa.

In particular, the trained (machine learning, ML) function (algorithm) of the methods and systems for use can be adapted by the methods and systems for training a function (ML algorithm, e.g., a neuronal network). Furthermore, the input data can comprise advantageous features and embodiments of the training input data, and vice versa. Furthermore, the output data can comprise advantageous features and embodiments of the output training data, and vice versa.

In general, a trained function mimics cognitive functions that humans associate with other human minds. In particular, by training based on training data the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Qlearning, genetic algorithms and/or association rules. In particular, a neural network can be a deep neural network such as a convolutional neural network. Furthermore, a neural network can be an adversarial network, a deep adversarial network and/or a generative adversarial network.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

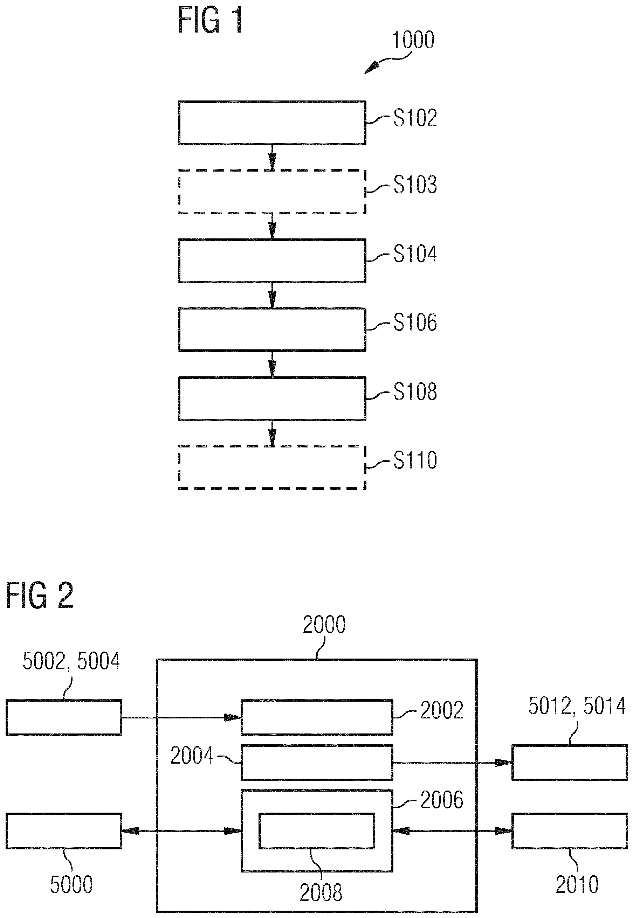

Fig. 1 illustrates a flow chart for using a trained function for three-dimensional segmentation of a lesion.

The flow chart illustrates steps for a computer-implemented method 1000 for providing output data (using a trained function) in order to locate a liver lesion in input data 5002, 5004.

The computer-implemented method 1000 comprises steps S102 (receiving input data), S104 (applying a trained function to the input data), S106 (generating output data) and S108 (providing the output data).

More specifically, the computer-implemented method 1000 comprises receiving in step S102, by a first computer interface, input data 5002, 5004. The computer-implemented method 1000 further comprises applying in step S104 a trained function 5000 to the input data 5002, 5004. The computer-implemented method 1000 further comprises generating in step S106, by the trained function 5000, output data 5012, 5014, wherein the output data is associated with the input data. The computer-implemented method 1000 further comprises providing in step S108, via a second computer interface, the output data 5012, 5014. The input data 5002, 5004 comprises a multi-phase three-dimensional, 3D, computed tomography, CT, imaging dataset of a liver lesion associated with a patient. The output data 5012, 5014 comprises a three-dimensional, 3D, segmentation data 5012; 5014 for locating the liver lesion in the input data 5002, 5004.

The segmentation data may include a segmentation map (output data 5014): the segmentation map identifies for each pixel or voxel of imaging data whether the respective pixel or voxel as part or not part of the segmented object. Other forms of segmentation data include splines or surfaces outlining the circumference of the segmented object. Yet another form of segmentation data includes a heat map (output data 5012). A heat map can include a probabilistic measure of a certain pixel or voxel of the imaging data being part or not part of the segmented object. Still another option for segmentation data includes bounding boxes or bounding spheres forming a part of output data 5012 or output data 5014.

In an example, the trained function 5000 for the computer-implemented method 1000 may comprise an encoder part 5006 for encoding each 2D slice of each phase P1, P2, ..., Pn from the multi-phase 3D CT imaging dataset (i.e., the input data 5002, 5004) into encoded feature information F1, F2, F3. The trained function 5000 may further comprise a spatial aggregation, SA, module 5010 for aggregating the encoded feature information F1, F2, F3 into spatially aggregated feature information Agg_F4.

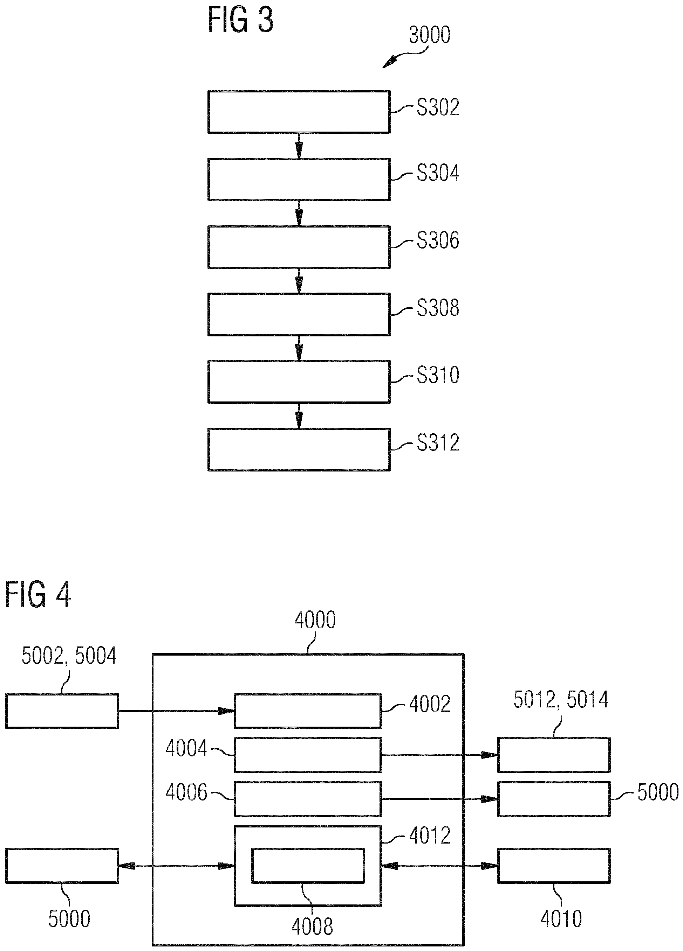

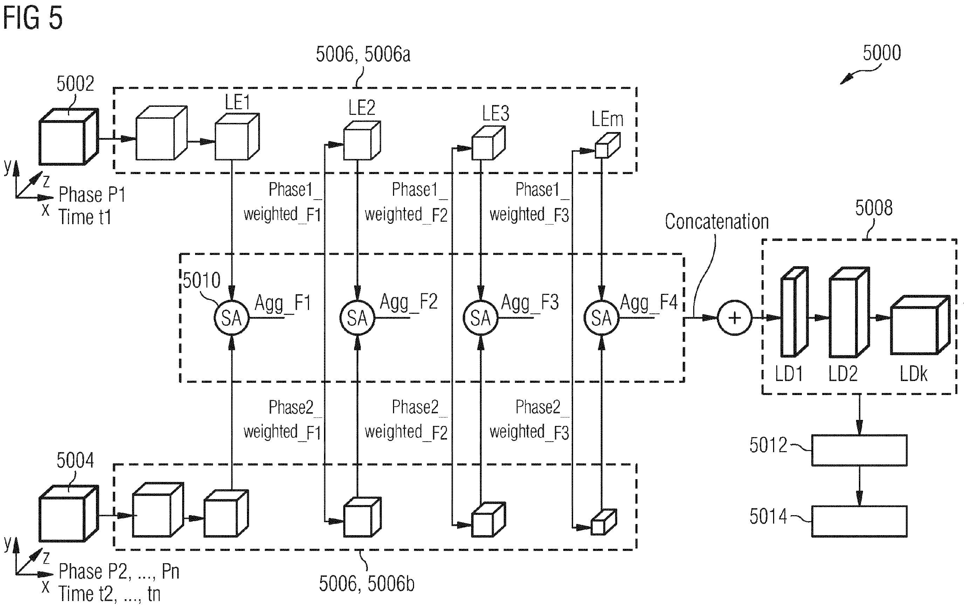

The trained function 5000 may comprise further features as described in the context of figs. 5-7. The trained function 5000 may be trained according to a computer-implemented method 3000 as described in the context of fig. 3. The trained function 5000 may be trained by a training system 4000 as described in the context of fig. 4.

Steps S103 (pre-processing of input data) and S110 (post processing of input data) are optional for the computer-implemented method 1000.

In an example, the computer-implemented method 1000 may comprise pre-processing of the input data in step S103 by a pre-processing algorithm. The pre-processing algorithm may comprise manually selecting, for each phase from the multi-phase 3D CT imaging dataset, a 2D slice, wherein a target lesion has the largest diameter, indicating a center or a diameter of the target lesion and receiving in step S102, via the first computer interface, the center or the diameter of the target lesion as a part of the input data 5002, 5004. Alternatively to manually selecting, for each phase from the multi-phase 3D CT imaging dataset, the 2D, slice, the pre-processing algorithm may comprise automatically detecting, by an additional trained function, a bounding-box or coordinates of a centroid associated with the target lesion on a 2D slice for each phase from the multi-phase 3D CT imaging dataset (the input data 5002, 5004) wherein the target lesion has the largest diameter, and receiving in step S102, via the first computer interface, by the trained function, the bounding-box or the coordinates of the centroid as a part of the input data 5002, 5004. The additional trained function comprises a deep neuronal network 100 such as a convolutional neuronal network 200 configured to receive the input data 5002, 5004 and provide the bounding box or the coordinates of the centroid to the trained function as a part of the input data 5002, 5004.

In an example, the pre-processing in step S103 may further comprise registering 2D slices of the multi-phase 3D CT imaging dataset (the input data 5002, 5004) associated with different phases P1, ..., Pn according to a mesh-based algorithm, or according to a point-cloud-based algorithm. The mesh-based algorithm may comprise providing a mesh having a plurality of interconnected data nodes representing a geometrical structure of a lesion within images of the multi-phase 3D CT imaging dataset (the input data 5002, 5004) for aligning or matching 2D slices associated with each phase P1, ..., Pn from the multi-phase 3D CT imaging dataset 5002, 5004 to the geometrical structure of the lesion. The point-cloud-based algorithm may comprise providing a point cloud comprising a plurality of data points representing the geometrical structure of the lesion within the images of the multi-phase 3D CT imaging dataset (the input data 5002, 5004) for identifying a geometrical transformation that aligns corresponding points in the point cloud for spatial correspondence between the 2D slices of every phase P1, ..., Pn from the multi-phase 3D CT imaging dataset (the input data 5002, 5004).

In an example, the computer-implemented method 1000 may further comprise post-processing the output data in step S110 by a post-processing algorithm. The post-processing algorithm may comprise rendering, by a computer processor 2008, the 3D segmentation data 5015; 5014. The post-processing algorithm may further comprise calculating, by the computer processor 2008, one or more parameters associated with the target lesion from the 3D segmentation data 5012; 5014. The one or more parameters may comprise any one of a largest diameter, a volume, a lesion type, a lesion's contour, a minimum and/or a maximum intensity of each lesion. The post-processing algorithm may further comprise providing the one or more parameters via the second computer interface 2004. The 3D segmentation data 5012, 5014 may comprises a three dimensional, 3D, heat map (output data 5012) and/or three dimensional segmentation mask (output data 5014) obtainable by generating, by the trained function, the three-dimensional, 3D, heatmap (output data 5012) and converting the generated 3D heatmap (output data 5012) into a 3D binary mask by applying a predefined threshold to the generated 3D heatmap (output data 5012) and identifying one or more connected components within the binary mask.

The post-processing algorithm may further comprise visual rendering the 3D heatmap (output data 5012) and the 3D heatmap (output data 5012) via the second computer interface 2004 and/or generating reports comprising one or more parameters. The visual rendering of the 3D heatmap (output data 5012) and the 3D heatmap (output data 5012) via the second computer interface 2004 and/or the reports comprising one or more parameters may assist a user (e.g., radiologist or a surgeon) in locating a lesion in the input data 5002, 5004.

Fig. 2 illustrates a system, i.e., computer system 2000, for using the trained function according to fig. 1.

The computer system 2000 comprises a first interface 2002 configured for receiving input data 5002, 5004. The computer system 2000 further comprises a second interface 2004, configured for providing output data 5012, 5014. The computer system 2000 further comprises a computation unit 2006 comprising a computer processor 2008 configured for applying a trained function 5000 trained by a machine learning algorithm to the input data 5002, 5004, wherein the output data 5012, 5014 is generated by the trained function 5000. The input data 5002, 5004 comprises a multi-phase three-dimensional, 3D, computed tomography, CT, imaging dataset of a liver lesion associated with a patient. The output data 5012, 5014 comprises a three-dimensional, 3D, segmentation data 5012; 5014 for locating the liver lesion in the input data 5002, 5004.

An exemplary implementation of the trained function 5000 for the computer system 2000 is described in the context of fig. 5.

In more general terms, the trained function 5000 for the computer system 2000 may comprise an encoder part 5006 for encoding each 2D slice of each phase P1, P2, ..., Pn from the multi-phase 3D CT imaging dataset (i.e., input data 5002, 5004) into encoded feature information F1, F2, F3. The trained function 5000 for the computer system 2000 may further comprise a spatial aggregation, SA, module 5010 for aggregating the encoded feature information F1, F2, F3 into spatially aggregated feature information Agg_F4. The trained function 5000 for the computer system 2000 may further comprise a decoder part 5008 for decoding the spatially aggregated feature information Agg_F4 into the 3D segmentation data 5012; 5014. The encoder part 5006 may comprises a first branch 5006a receiving 2D slices of the input data 5002 associated with a reference phase P1 and a second branch 5006b receiving 2D slices of the input data 5004 associated with one or more remaining phases P2, ..., Pn, each of the first branch 5006a and the second branch 5006b providing the encoded feature information F1, F2, F3 to the SA module for aggregation. Each of the first branch 5006a and the second branch 5006b of the encoder part 5006 may comprise multiple stacked layers LE1, ..., Lem that may be implemented as layers 110-113 of a neuronal network 100 or layers 210-214 of a neuronal network 200. The multiple stacked layers LE1, ..., Lem may provide the encoded feature information F1, F2, F3 to the SA module 5010 for aggregation into the spatially aggregated feature information Agg_F4.

In an example, the computation unit 2006 of the computer system 2000 may further be configured to pre-process the input data 5002, 5004 according to a pre-processing algorithm. The pre-processing algorithm may comprise manually selecting, for each phase from the multi-phase 3D CT imaging dataset, a 2D slice, wherein a target lesion has the largest diameter, indicating a center or a diameter of the target lesion and receiving in step S102, via the first computer interface, the center or the diameter of the target lesion as a part of the input data 5002, 5004. Alternatively to manually selecting, for each phase from the multi-phase 3D CT imaging dataset, the 2D, slice, the pre-processing algorithm may comprise automatically detecting, by an additional trained function, a bounding-box or coordinates of a centroid associated with the target lesion on a 2D slice for each phase from the multi-phase 3D CT imaging dataset (the input data 5002, 5004) wherein the target lesion has the largest diameter, and receiving in step S102, via the first computer interface, by the trained function, the bounding-box or the coordinates of the centroid as a part of the input data 5002, 5004. The additional trained function comprises a deep neuronal network 100 such as a convolutional neuronal network 200 configured to receive the input data 5002, 5004 and provide the bounding box or the coordinates of the centroid to the trained function as a part of the input data 5002, 5004.

The pre-processing algorithm may further comprise registering 2D slices of the multi-phase 3D CT imaging dataset (the input data 5002, 5004) associated with different phases P1, ..., Pn according to a mesh-based algorithm, or according to a point-cloud-based algorithm. The mesh-based algorithm may comprise providing a mesh having a plurality of interconnected data nodes representing a geometrical structure of a lesion within images of the multi-phase 3D CT imaging dataset (the input data 5002, 5004) for aligning or matching 2D slices associated with each phase P1, ..., Pn from the multi-phase 3D CT imaging dataset (the input data 5002, 5004)to the geometrical structure of the lesion. The point-cloud-based algorithm may comprise providing a point cloud comprising a plurality of data points representing the geometrical structure of the lesion within the images of the multi-phase 3D CT imaging dataset (the input data 5002, 5004) for identifying a geometrical transformation that aligns corresponding points in the point cloud for spatial correspondence between the 2D slices of every phase P1, ..., Pn from the multi-phase 3D CT imaging dataset (the input data 5002, 5004).

In an example, computation unit 2006 of the computer system 2000 may be configured to post-process the output data 5012, 5014 according to a post-processing algorithm. The post-processing algorithm may comprise rendering, by a computer processor 2008, the 3D segmentation data 5015; 5014. The post-processing algorithm may further comprise calculating, by the computer processor 2008, one or more parameters associated with the target lesion from the 3D segmentation data 5012; 5014. The one or more parameters may comprise any one of a largest diameter, a volume, a lesion type, a lesion's contour, a minimum and/or a maximum intensity of each lesion. The post-processing algorithm may further comprise providing the one or more parameters via the second computer interface 2004. The 3D segmentation data 5012, 5014 may comprises a three-dimensional, 3D, heat map (output data 5012) and/or three dimensional segmentation mask (output data 5014) obtainable by generating, by the trained function, the three-dimensional, 3D, heatmap (output data 5012) and converting the generated 3D heatmap (output data 5012) into a 3D binary mask by applying a predefined threshold to the generated 3D heatmap (output data 5012) and identifying one or more connected components within the binary mask.

The post-processing algorithm may further comprise visual rendering the 3D heatmap (output data 5012) and the 3D heatmap (output data 5012) via the second computer interface 2004 and/or generating reports comprising one or more parameters. The visual rendering of the 3D heatmap (output data 5012) and the 3D heatmap (output data 5012) via the second computer interface 2004 and/or the reports comprising one or more parameters may assist a user (e.g., radiologist or a surgeon) in locating a lesion in the input data 5002, 5004.

Fig. 3 illustrates a flow chart for training a training function for use according to fig. 1.

The flow chart illustrates a computer-implemented method 3000 for training a training function 500. The computer-implemented method 3000 for providing a trained function 5000 comprises steps S302 (receiving training input data), S304 (providing the training input data to a training function), S306 (embedding the training input data), S308 (receiving output training data), S310 (generating a trained function), and S312 (providing the trained function for use).

The computer implemented method 3000 for providing a trained function 5000 comprises receiving, at step S302, by a first training computer interface 4002, training input data 5002, 500). The computer implemented method 3000 further comprises providing, at step S304, by a computer processor 4008, the training input data to a training function 5000. The computer implemented method 3000 further comprises embedding, at step S306, by the training function 5000, the training input data 5002, 5004. The computer implemented method 3000 further comprises receiving, at step S308, via a second training computer interface 4004, training output data 5012, 5014, wherein the training output data is associated with the training input data. The computer implemented method 3000 further comprises generating, at step S310, by the computer processor 4008, a trained function comprising embedded training input data. The computer implemented method 3000 further comprises providing, at step S312, via a third training computer interface 4006, the trained function 5000 for use. The training input data 5002, 5004 comprises one or more multi-phase three-dimensional, 3D, computed tomography, CT, imaging datasets of the input data 5002, 5004 of one or more liver lesions, wherein each imaging dataset is associated with a patient.

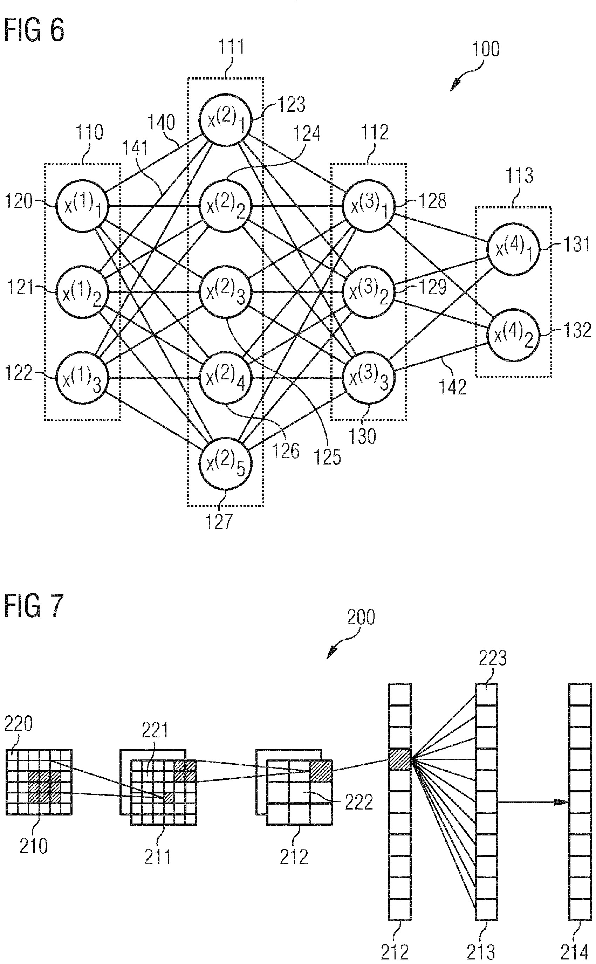

An exemplary implementation of a trained function 5000 is described in the context of figs. 5-7. However, other implementations of the trained function 5000 are within the scope. For example, a trained function 5000 can comprise a neural network, a support vector machine, a decision tree and/or a Bayesian network, and/or the trained function can be based on k-means clustering, Qlearning, genetic algorithms and/or association rules. As described in the context of figs. 5 and 6, the neural network for the trained function 5000 can be a deep neural network. As described in the context of figs. 5 and 6, the neural network for the trained function 5000 can be a convolutional (deep) neural network. Other examples of a neuronal network may include a neural network that can be an adversarial network, a deep adversarial network and/or a generative adversarial network.

In an example, the trained function 5000 for the computer-implemented method 3000, may comprise an encoder part 5006 for encoding each 2D slice of each phase P1, P2, ..., Pn from the imaging datasets of the input data 5002, 5004 into encoded feature information F1, F2, F3. The trained function 5000 may further comprise a spatial aggregation, SA, module 5010 for aggregating the encoded feature information F1, F2, F3 into spatially aggregated feature information Agg_F4, and a decoder part 5008 for decoding the spatially aggregated feature information Agg_F4 into the 3D segmentation data 5012; 5014. The encoder part 5006 may comprise a first branch 5006a receiving 2D slices of the imaging datasets of the input data 5002 associated with a reference phase P1 and a second branch 5006b receiving 2D slices of the imaging datasets (input data 5004) associated with one or more remaining phases P2, ..., Pn, each of the first branch 5006a and the second branch 5006b providing the encoded feature information F1, F2, F3 to the SA module for aggregation. Each of the first branch 5006a and the second branch 5006b of the encoder part 5006 may comprise multiple stacked layers LE1, ..., Lem that may be implemented by layers 110-113 of a deep neuronal network 100 or layers 210-214 of a convolutional neuronal network 200, wherein the multiple stacked layers LE1, ..., Lem provide the encoded feature information (F1, F2, F3) to the SA module (5010) for aggregation into the spatially aggregated feature information Agg_F4.

In an example, the computer-implemented method 3000 may further comprise detecting one or more regions of interest, ROIs, for each lesion within each image of the multi-phase 3D CT imaging dataset (the input data 5002, 5004). The detecting may comprise determining a centre of mass or centroid of each lesion. the computer-implemented method 3000 may further comprise cropping each image of the multi-phase 3D CT imaging dataset (the input data 5002, 5004) around the centre of mass or centroid. the computer-implemented method 3000 may further comprise encoding cropped images by the encoder part 5006. the computer-implemented method 3000 may further comprise matching the centres of mass or centroids across the the multi-phase 3D CT imaging dataset to establish a representation of the lesion's position in three-dimensional, 3D, space. the computer-implemented method 3000 may further comprise aggregating, by the SA module 5010, encoded feature information F1, F2, F3 into spatially aggregated feature information Agg_F4. the computer-implemented method 3000 may further comprise generating, by the decoder part 5008 decoding the spatially aggregated feature information Agg_F4, a three-dimensional, 3D, heat map (output data 5012), wherein the 3D heat map is a 3D graphical representation indicating spatial location of each lesion across the multi-phase 3D CT imaging dataset. the computer-implemented method 3000 may further comprise generating, by applying a threshold to the generated 3D heatmap, a 3D segmentation mask (output data 5014).

In an example, the computer-implemented method 3000 may further comprise training an additional function. The additional function may comprise a deep neuronal network 100; 200 to provide an additional trained function. The additional function may be trained by embedding additional training data associated with a plurality of bounding-boxes or coordinates of a plurality of centroids each associated with a lesion present in the training input data 5002, 5004 provided to the additional function together with the plurality of bounding-boxes or coordinates of the plurality of centroids via the first training computer interface 4002. The additional trained function may be trained to generate output data being a bounding-box or coordinates of a centroid associated with a target lesion. The additional trained function may be chained with the trained function of the computer-implemented method 1000 and/or computer-implemented method 3000 such that the output data generated by the additional trained function may be provided as an additional input data in addition to the input data 5002, 5004 provided to the trained function of the computer-implemented method 1000 and/or computer-implemented method 3000 for automatic detection of a bounding-box or coordinates of a centroid associated with a target lesion present in the input data 5002, 5004.

In the context of the current disclosure input data 5002, 5004 and training input data 5002, 5004 may comprise identical data. For example, when a function is trained using training input data 5002, 5004, it may be evaluated by using input data 5002, 5004 that is identical to (one of the) training input data 5002, 5004 used for training.

Input data 5002, 5004 may have identical or similar structure and type to the training input data 5002, 5004 (i.e., both input data 5002, 5004 and training input data 5002, 5004 being a multi-phase three-dimensional, 3D, computed tomography, CT, imaging dataset of a liver lesion associated with a patient). Input data 5002, 5004 may be used for training, and thus, may become training input data 5002, 5004.

In an example, the computer-implemented method 3000 may further comprise receiving, by the first training computer interface, a three-dimensional, 3D, ground truth segmentation mask corresponding to the 3D heatmap. The computer-implemented method 3000 may further comprise calculating a weighted loss function by integrating a cross-entropy loss component and a dice loss component, wherein the cross-entropy loss component may quantify dissimilarity between predicted and actual probability distributions of class labels, wherein the actual probability distribution may be a binary vector representing the true class, where the probability is one for the correct class representing a lesion and zero for other classes that do not represent a lesion. The dice loss component may quantify the spatial overlap between predicted and the ground truth segmentation masks. The computer-implemented method 3000 may further comprise iteratively updating one or more hyperparameters of the trained function based on the calculated weighted loss function, wherein the one or more hyperparameters may comprise any one of or a combination thereof a type of optimizer, a learning rate and/or a regularization weight. The computer-implemented method 3000 may further comprise generating the trained function comprising the one or more hyperparameters.

In an example, the computer-implemented method 3000 may further comprise evaluating the trained function according to an evaluation algorithm. The evaluation algorithm may comprise receiving, via the first training computer interface, the training input data by the trained function 5000. The evaluation algorithm may further comprise The evaluation algorithm may further comprise generating, by the trained function 5000, a 3D heatmap. The evaluation algorithm may further comprise generating a binary mask from the generated 3D heatmap by applying a predetermined threshold to the 3D heatmap. The evaluation algorithm may further comprise identifying a 3D segmentation mask that aligns with the center of the lesion based on identifying connected components within the binary mask by: identifying spatial proximity between pixels of the binary mask, assigning one or more labels to the one or more connected components for distinguishing the one or more components, generating an output representation comprising the labelled connected components. The evaluation algorithm may further comprise generating the identified 3D segmentation mask and providing the generated 3D segmentation mask via the second training computer interface 4004 as training output data 5014. The evaluation algorithm may further comprise providing, via the first training computer interface 4002, a comparative ground truth segmentation mask associated with the training input data. The evaluation algorithm may further comprise comparing the generated 3D segmentation mask with the comparative ground truth segmentation mask, wherein the comparative ground truth segmentation mask may be an annotated set of binary images that serves as a reference delineating the true boundaries or labels of the connected components. The evaluation algorithm may further comprise calculating an evaluation metric by comparing the generated 3D segmentation mask and the comparative ground truth segmentation mask. The evaluation metric may comprise one or more performance characteristics of the trained function. The evaluation algorithm may further comprise providing the calculated evaluation metric via the second 4004 or the third training computer interface 4006.

Training output data 5012, 5014 may be identical or similar to output data 5012, 5014 in that both data may have the same data type and structure (i.e., a three-dimensional, 3D, segmentation data). Training output data 5012, 5014 may comprise additional data associated with training a function, and, optionally, evaluating the trained function.

Computer readable instructions 2010, 4010 described in the context of figs. 2 and 4 may be used to provide a computer program product. The computer program product of the disclosure comprises computer readable instructions 2010, 4010 which, when executed by a computer processor, cause the computer processor to carry out the method as described in the context of figs. 1 and 3.

Fig. 4 illustrates a system for training the training function of fig. 3.

The training system 4000 illustrated in fig. 4 comprises a first training computer interface 4002 configured for receiving training input data 5002, 5004. The training system 4000 a training computation unit 4012. The computation unit 4012 comprise a computer processor 4008 configured for training a training function 5000 by providing the training input data 5002, 5004 to the training function 5000 for embedding, by the training function, the training input data. The computer processor 4008 is further configured for generating the trained function 5000 comprising the embedded training data. The training system 4000 further comprises a second training computer interface 4004 configured for providing output data 5012, 5014, wherein the output data is associated with the input data. The training system 4000 a third training computer interface 4006 configured for providing the trained function 5000 for use, wherein the training input data 5002, 5004 comprises one or more multi-phase three-dimensional, 3D, computed tomography, CT, imaging datasets of the input data 5002, 5004 of one or more liver lesions, wherein each imaging dataset is associated with a patient.

The trained function 5000 generated by the training system 4000 described in the context of fig. 4 may be used for the computer-implemented methods as described in the context of figs. 1 and 3.

The trained function 5000 generated in accordance with the computer-implemented method 3000 described in the context of fig. 3 may be generated by the training system 4000 described in the context of fig. 4.

The trained function described in the context of figs. 3 and 4 may be used by the computer implemented method 1000 described in the context of fig. 1 and by the computer system 2000 described in the context of fig. 2.

Fig. 5 illustrates an encoder-decoder architecture with an integrated spatial aggregator module for three-dimensional segmentation of figs. 1-4.

The encoder-decoder architecture illustrated in fig. 5 is an exemplary architecture for a trained function 5000 for the computer-implemented methods 1000 and 3000 described in the context of figs. 1 and 3, respectively, and for the systems 2000 and 4000 described in the context of figs. 2 and 4, respectively.

In an example, the trained function 5000 for the computer-implemented method 1000 may comprise an encoder part 5006 for encoding each 2D slice of each phase P1, P2, ..., Pn from the multi-phase 3D CT imaging dataset (i.e., the input data 5002, 5004) into encoded feature information F1, F2, F3. The trained function 5000 may further comprise a spatial aggregation, SA, module 5010 for aggregating the encoded feature information F1, F2, F3 into spatially aggregated feature information Agg_F4.

The trained function 5000 may further comprise a decoder part 5008 for decoding the spatially aggregated feature information Agg_F4 into the 3D segmentation data 5012; 5014. The encoder part 5006 may comprise a first branch 5006a receiving 2D slices of the input data 5002 associated with a reference phase P1 and a second branch 5006b receiving 2D slices of the input data 5004 associated with one or more remaining phases P2, ..., Pn, each of the first branch 5006a and the second branch 5006b providing the encoded feature information F1, F2, F3 to the SA module for aggregation. Each of the first branch 5006a and the second branch 5006b of the encoder part 5006 may comprise multiple stacked layers LE1, ..., LEm of a neuronal network providing the encoded feature information F1, F2, F3 to the SA module 5010 for aggregation into the spatially aggregated feature information Agg_F4.

The first encoder branch 5006a and the second encoder branch 5006b may process input data 5002, 5004 through a series of layers LE1, ..., LEm to reduce the dimensionality of the input data 5002, 5004, transforming the input data 5002, 5004 into a compact representation referred to as a feature embedding or encoded feature information F1, F2, F3. This embedding or encoded feature information F1, F2, F3 may capture characteristics of the input data 5002, 5004 in a lower-dimensional space. By systematically compressing the input data 5002, 5004 in this way, the first encoder branch 5006a and second encoder branch 5006b facilitate the extraction of patterns associated with a lesion (e.g., contour, texture, shape, diameter, etc.) that are crucial for subsequent tasks, such as segmentation of input data 5002, 5004 for locating a lesion in the input data 5002, 5004.

In an example, multiple stacked layers LE1, ..., LEm of the first branch 5006a and/or the second branch 5006b as illustrated in fig. 5 may be implemented as multiple stacked layers 110-113 of a deep neuronal network 100 as described in the context of fig. 6.

In another example, multiple stacked layers LE1, ..., LEm of the first branch 5006a and the second branch 5006b as illustrated in fig. 5 may be implemented as multiple stacked layers 210-214 of a convolutional neuronal network 200 as described in the context of fig. 7.

More specifically, aggregated feature information Agg_F4 may be obtained from a plurality of aggregated features (e.g., Agg_F1, Agg_F2, Agg_F3, and so on). Each aggregated feature Agg_F1, Agg_F2, Agg_F3, and so on may be obtained by SA module 5010 by aggregating encoded feature information F1, F2, F3, and so on provided by each layer of the encoder part 5006, LE1, LE2, ..., LEm, respectively, where m is an integer number.

For example, feature Agg_F1 may be obtained by SA module 5010 by aggregating encoded feature information F1 from a first layer LE1 of the first encoder branch 5006a and from a first layer LE1 of the second encoder branch 5006b. Encoded feature information F1 from a first layer LE1 of the first encoder branch 5006a may be obtained by encoding (or embedding) input data 5002, wherein the input data 5002 is obtained at a time t1 for a reference phase P1. Encoded feature information F1 from a first layer LE1 of the second encoder branch 5006b may be obtained by encoding (or embedding) input data 5004, wherein the input data 5004 is obtained at time t2, ..., tn for remaining phases P2, ..., Pn, respectively, wherein n is an integer number. Thus, encoded feature information F1 from a first layer LE1 of the second encoder branch 5006b may be a plurality of encoded feature information F1, each encoded feature information F1 being obtained from input data 5004 associated with each phase from the remaining phases P2, ..., Pn. Each phase P1, P2, ..., Pn in the context of fig. 5 is associated with a time t1, t2, ..., tn at which input data 5002, 5004 is recorded when a contrast agent is present in the blood of a patient. In the same way as obtaining encoded feature information F1 by the first layer LE1, encoded feature information F2, F3, etc. may be obtained by a corresponding layer LE2, LE3, etc. of the encoder part 5006, respectively.

Each encoded feature information F1 provided by the first layer LE1 of the encoder part 5006 may be aggregated by SA module 5010 into spatially aggregated feature information Agg_F1. Each encoded feature information F2 provided by the second layer LE2 of the encoder part 5006 may be aggregated by SA module 5010 into spatially aggregated feature information Agg_F2, and so on until the last layer LEm of the encoder part 5006.

Encoded feature information F1 extracted by the first layer LE1 of the encoder part 5006 may be weighted based on the phase, e.g., Phase1_weighted_F1, Phase2_weighted_F1, etc. until the last phase Pn. Encoded feature information F2 extracted by the second layer LE2 of the encoder part 5006 may be weighted based on the phase, e.g., Phase1_weighted_F2, Phase2_weighted_F2, etc. until the last phase Pn; and so on until the last layer LEm of the encoder part generating the last encoded feature information.

Spatially aggregated feature information Agg_F4 obtained by SA module 5010 from the encoder part 5006 (from the last layer LEm) may be provided to a decoder part 5008 for decoding.

The decoder part 5008 may be used for upscaling the condensed or, in other terms, spatially aggregated feature information Agg_F4. The decoder part 5008 may be used to reconstruct or generate output data 5012, 5014 from the aggregated feature information Agg_F4, where output data 5012, 5014 may match the original input data 5002, 5004 size or a specified target (e.g., a ROI). The decoder part 5008 may progressively increase the spatial dimensions of the feature maps (aggregated feature information Agg_F4) using operations such as transposed convolutions (also referred to as deconvolutions), upsampling, or unpooling layers. Throughout this process, the decoder part 5008 may combine the upsampled features with corresponding feature maps from the encoder part 5006 via skip connections, which result in preserving spatial information that might have been lost during downsampling. The decoder part 5008 may reconstruct the input data 5002, 5004 from the compressed feature representations (aggregated feature information Agg_F4) for further tasks such as segmentation of input data 5002, 5004 for locating a lesion in the input data 5002, 5004.

In an example, the decoder part 5008 may be implemented by multiple stacked layers LD1, ..., LDk, as illustrated in fig. 5, where k is an integer number.

In an example, multiple stacked layers LD1, ..., LDk, as illustrated in fig. 5, may be implemented as multiple stacked layers 110-113 of a deep neuronal network 100 as described in the context of fig. 6.

In another example, multiple stacked layers LD1, ..., LDk, as illustrated in fig. 5, may be implemented as multiple stacked layers 210-214 of a convolutional neuronal network 200 as described in the context of fig. 7.

As described above, the encoded feature information F1, F2, F3 in the context of fig. 5 is associated with phase-specific features from input data 5002, 5004. The input data 5002, 5004 in the context of fig. 5 is a three-dimensional data indicated by axes x, y, z, each axis x, y, z being associated with a three-dimensional space of an organ (liver). The data may be obtained at time points t1, t2, ..., tn. Each time point t1, t2, ..., tn may be associated with a phase P1, P2, ..., Pn, respectively, wherein n is an integer number. A phase in the context of CT or MRI imaging may be associated with a contrast agent providing contrast information in each phase P1, P2, ..., Pn for identifying a lesion.

The spatial aggregation module 5010 may aggregate encoded feature information F1, F2, F3 at different stages (i.e., at layers L1, ..., LEm) of the encoder part 5006 to calculate aggregated feature information Agg_F4 that can leverage cross-phase information, i.e., account for encoded feature information F1, F2, F3, etc. from each phase P1, .., Pn. Stated differently, information from each phase P1, P2, ..., Pn may advantageously contribute to each feature F1, F2, F3 thereby contributing to the aggregated feature information Agg_F4 resulting in that aggregated feature information Agg_F4 may enhance accuracy of segmenting a lesion. Enhanced accuracy of segmenting a lesion, in turn, may enhance the accuracy of locating a lesion.

In an example, encoder part 5006 may be implemented by layers of a neuronal networks described in the context of figs. 6 and 7. There may be other implementations of the encoder part 5006 such as by a pre-trained neuronal networks (e.g., a U-Net, a ResNet, or any pre-trained feature extractor model).

The decoder part 5008 may take the combined aggregated representation of the input patches (input data 5002, 5004) represented by the aggregated feature information Agg_F4 and may transform the aggregated feature information Agg_F4 into output data 5012, 5014. The output data 5012, 5014 may comprise a 3D heatmap for locating a lesion.

Output data 5012, i.e., the 3D heatmap, may be transformed to a binary 3D mask by performing a thresholding.

A connected component analysis may be performed to extract the binary 3D mask of the lesion of reference.

In an example, a dynamic thresholding may be implemented based on a radiologist providing initial key points of the lesion in the manual selection.

Advantageously, heatmaps generated by an alternative trained function (e.g., as the ones disclosed in NPL1-NPL4) may be aggregated with the multi-phase guided neural network segmentation heatmaps (i.e., output data 5012) using heatmaps weighted summation by, e.g., thresholding and performing binary masks logical operators.

Advantageously, output data 5012, 5014 may be post-processed according to a post processing algorithm. In an example, the post processing algorithm may be used to obtain lesion's parameters such as the largest diameter, the volume, minimum and maximum intensity of each target tumor in the output data 5012, 5014.

In an example, the post processing algorithm may be used to generate a 3D rendering from the output data 5012, 5014. The 3D rendering may visually represent a target lesion. The 3D rendering may comprise color-coded rendering according to the lesion type.

Advantageously, the post processing algorithm may be used to display a 3D contour of the target lesion of the actual study overlaid on any of the phase's P1 images. In other words, the 3D rendering may comprise displaying a 3D contour of the target lesion of the actual study overlaid on any of the phase's P1 images.

Advantageously, the disclosure may allow to integrate multi-phase information from 3D contrast studies of an organ, based on a cross-pixel aggregation mechanism into a deep learning encoder-decoder architecture such as the one illustrated in fig. 5, enabling multi-phase CT scans to collaboratively guide the segmentation of a target lesion onto a reference phase P1. The encoder-decoder architecture may allow for automatically enhancing segmentation accuracy thereby improving locating of a lesion.

Advantageously, the 3D rendering of the Output data 5012, 5014 may guide a user (e.g., surgeon) for locating a lesion.

Subsequently, advantageously, output data 5012, 5014 may be exported to the report further guiding a user for locating a lesion or making a decision based on one or more parameters of the lesion identified in the report.

A single-phase imaging, such as disclosed in the prior art, can present challenges when dealing with tumors that lack clear outlines or exhibit complex characteristics. These methods don't optimally exploit the information available across multiple phases, especially in scenarios where certain features might be more visible in one phase than another.

Advantageously, the architecture described in the context of fig. 5 may allow to perform guided segmentation for the methods and systems described in the context of figs. 1-4. Advantageously, said guided segmentation may use information from all available phases, P1, ..., Pn to guide and refine the segmentation process, that may mimic and improve the manual method steps of radiological practices. The methods and systems of the current disclosure ensure that the segmentation process aligns closely with clinical expertise (mimics the practice) and provides further enhancement and guidance to radiologists when segmenting the lesions automatically.

Due to the SA 5010, the architecture in the context of fig. 5 can provide effective capture of spatial information from each phase, P1, ..., Pn. Moreover, the implementation of cross-pixel aggregation by the SA module 5010 can inherently minimize redundancy during cross-phase aggregation, as compared to known attention/aggregation mechanisms.

The architecture in the context of fig. 5 may be particularly advantageous for automatic detection of liver lesions.

Enabling automatic detection of lesions by an additional trained function may allow the systems and methods of the current disclosure to work fully automatically and thus not having to wait for user interaction (e.g., when a radiologist provides input). This means that, advantageously, all lesions may be automatically identified and aligned with their counterparts in other phases by the additional trained function prior to automatic segmentation by the trained function.

Mesh-based or point-cloud-based registration may be particularly advantageous since it may offer a higher level of alignment precision that may, in turn, be advantageous for effective cross-pixel aggregation by the SA module 5010.

Fig. 6 illustrates a deep neuronal network for the encoder-decoder neural network architecture of fig. 5.

In general, and in the context of the disclosure, alternative terms for a deep neuronal network 100 may also be "artificial neural network", "neural network", "artificial neural net" or "neural net".

The deep neural network 100 comprises nodes 120, ..., 132 and edges 140, ..., 142, wherein each edge 140, ..., 142 is a directed connection from a first node 120, ... , 132 to a second node 120, ..., 132. In general, the first node 120, ..., 132 and the second node 120, ..., 132 are different nodes 120, ..., 132, it is also possible that the first node 120, ..., 132 and the second node 120, ..., 132 are identical. For example, in fig. 6 the edge 140 is a directed connection from the node 120 to the node 123, and the edge 142 is a directed connection from the node 130 to the node 132. An edge 140, ... , 142 from a first node 120, ..., 132 to a second node 120, ..., 132 is also denoted as "ingoing edge" for the second node 120, ..., 132 and as "outgoing edge" for the first node 120, ..., 132.

In this embodiment, the nodes 120, ..., 132 of the deep neural network 100 can be arranged in layers 110, ..., 113, wherein the layers can comprise an intrinsic order introduced by the edges 140, ..., 142 between the nodes 120, ..., 132. In particular, edges 140, ..., 142 can exist only between neighbouring layers of nodes. In the displayed embodiment, there is an input layer 110 comprising only nodes 120, ..., 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ..., 122 within the input layer 110 usually relates to the number of input values of the neural network, and the number of nodes 131, 132 within the output layer 113 usually relates to the number of output values of the neural network.

In particular, a (real) number can be assigned as a value to every node 120, ..., 132 of the neural network 100. Here, $x^{(n)}_i$ denotes the value of the i-th node 120, ..., 132 of the n-th layer 110, ..., 113. The values of the nodes 120, ..., 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, $w^{(m,n)}_{i,j}$ denotes the weight of the edge between the i-th node 120, ..., 132 of the m-th layer 110, ..., 113 and the j-th node 120, ..., 132 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ..., 132 of the (n+1)-th layer 110, ..., 113 can be calculated based on the values of the nodes 120, ..., 132 of the n-th layer 110, ..., 113 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the edges, the neural network 100 has to be trained using training data. In particular, training data comprises training input data and training output data (denoted as $t_i$). For a training step, the neural network 100 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $b^{(n+1)}_j$, if the (n+1)-th layer is not the output layer, and

$$\delta^{(n)}_j = \left(x^{(n+1)}_k - t^{(n+1)}_j\right) \cdot f'\left(\sum_i x^{(n)}_i \cdot w^{(n)}_{i,j}\right)$$

if the (n+1)-th layer is the output layer 113, wherein f' is the first derivative of the activation function, and $y^{(n+1)}_j$ is the comparison training value for the j-th node of the output layer 113.

A deep neuronal network 100 described in the context of fig. 6 may be implemented as a convolutional network 200 illustrated in fig. 7.

Fig. 7 illustrates a convolutional neuronal network for the encoder-decoder neural network architecture of fig. 5.

In general, a convolution layer in the context of convolutional network 200 works by applying a set of learnable filters (or kernels) across the input data (e.g., input data 5002, 5004), such as an image (e.g., 2D slice of input data 5002, 5004), to produce a feature map (represented by encoded feature information F1, F2, F3). Each filter may be designed to detect specific types of features at various locations in the input data 5002, 5004. As the filter slides (or convolves) across the input data 5002, 5004, it may perform element-wise multiplication with the part of the 2D slice of input data 5002, 5004 it covers, and the results may be summed up to produce a single pixel in the output feature map (represented by encoded feature information F1, F2, F3).

This process may allow the convolutional neuronal network 200 to capture spatial hierarchies of features (i.e., encoded feature information F1, F2, F3) such as edges, textures, etc. by learning filters that activate in response to specific patterns in the input data 5002, 5004. The encoded feature information F1, F2, F3 may be associated with the shape, size, edges, textures of a lesion and/or healthy tissue (of a liver).

The convolutional network 200 illustrated in fig. 7 is illustrated for a two-dimensional case, i.e., for a 2D slice of input data 5002, 5004.

In the context of the input data 5002, 5004 comprising a multi-phase three-dimensional, 3D, imaging dataset of the input data 5002, 5004, the convolutional neural network 200 may be adapted to the three-dimensional case, 3D CNN, to embed the 3D imaging dataset of the input data 5002, 5004.

Adapting a two-dimensional convolutional neural network, 2D CNN, to a three-dimensional, 3D CNN may involve the following modifications to account for the extra dimension in the input data 5002, 5004.

2D CNNs may receive inputs with dimensions (e.g., height, width, channels), where "channels", e.g., may represent color channels in a 2D slice of input data 5002, 5004.

3D CNNs may require inputs with dimensions (e.g., depth, height, width, channels) for volumetric data, i.e., input data 5002, 5004 for reference phase P1; or (time, height, width, channels) for sequential image data, i.e., input data 5002, 5004 for remaining phases P2, ..., Pn, adding an extra dimension for depth or temporal sequence.

Adapting 2D CNN to 3D CNN may further involve switching from 2D convolutional layers 211 as illustrated in fig. 7, which perform convolutions over a 2D grid (height x width), to 3D convolutional layers adapted to the input data 5002, 5004, which perform convolutions over a 3D grid (depth x height x width). The switching may involve specifying the depth in addition to the height and width of the convolutional kernels Kk.

Adapting 2D CNN to 3D CNN may further involve replacing 2D pooling layers 212 as illustrated in fig. 7, which reduce the spatial dimensions (height x width) of the feature maps, with 3D pooling layers, which reduce the spatial dimensions along depth, height, and width.

Adapting 2D CNN to 3D CNN may further involve adjusting the kernel Kk sizes for convolutional and pooling layers to include the third dimension. For example, use a kernel Kk size of (3, 3, 3) in a 3D CNN instead of (3, 3) in a 2D CNN.

Adapting 2D CNN to 3D CNN may further involve adjustment in other layers.

The transition from convolutional layers to fully connected layers may remain the same for 2D CNN and 3D CNN. The activation function can generally be adapted from 2D CNN to 3D CNN without substantive changes.

Adapting 2D CNN to 3D CNN may further involve adjusting hyperparameters (e.g., kernel sizes, numbers of filters, and/or alike).

For example, switching from a 2D to a 3D CNNs may mainly involve replacing 2D-specific operations with their 3D counterparts. For example, changing a layer from 2D to 3D may be described by a pseudo-code as follows. The pseudo-code for implementing a 2D CNN Layer may be as follows: layer_2d = Conv2D(filters=32, kernel_size=(3, 3), activation='relu').

The pseudo-code for implementing a 3D CNN Layer may be as follows: layer_3d = Conv3D(filters=32, kernel_size=(3, 3, 3), activation='relu').

In more general terms, adapting a 2D CNN to a 3D CNN may involve adjusting the architecture to operate over an additional dimension.

In another example, the convolutional neural network 200 illustrated in fig. 7 may be adapted to the 3D imaging dataset of the input data 5002, 5004, as, for example, described in NPL1 or NPL3. In case the convolutional neuronal network 200 is adapted to the 3D imaging datasets as described in NPL1, the convolutional neural network 200 may be implemented as a U-Net. In case the convolutional neuronal network 200 is adapted to the 3D imaging datasets as described in NPL3, the convolutional neural network 200 may be implemented as a Res-Net.

In fig. 7, a 2D CNN is illustrated adapted for encoding a 2D slice of the input data 5002, 5004. The convolutional neural network 200 in fig. 7 comprises an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ..., 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as $x^{(n)}[i,j]$. However,

the arrangement of the nodes 220, ..., 224 of one layer 210, ... , 214 does not have an effect on the calculations executed within the convolutional neural network 200 as such, since these are given solely by the structure and the weights of the edges.

In particular, a convolutional layer 211 may be characterized by the structure and the weights of the incoming edges forming a convolution operation based on a certain number of kernels. In particular, the structure and the weights of the incoming edges may be chosen such that the values x(n)k of the nodes 221 of the convolutional layer 211 may be calculated as a convolution $x^{(n)}k = Kk * x^{(n-1)}$ based on the values $x^{(n-1)}$ of the nodes 220 of the preceding layer 210, where the convolution * may be defined for a 2D slice of input data 5002, 5004 as

$$x_k^{(n)}[i,j] = (K_k * x^{(n-1)})[i,j] = \sum_{i'} \sum_{j'} K_k[i',j'] \cdot x^{(n-1)}[i-i', j-j'] .$$

Here the k-th kernel Kk is a d-dimensional matrix (in this embodiment a two-dimensional matrix), which is usually small compared to the number of nodes 220, ..., 224 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 220, ..., 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a dimensional matrix, using a plurality of kernels can be interpreted as adding a further dimension (denoted as "depth" dimension), so that the nodes 221 of the convolutional layer 211 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 211 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a nonlinear pooling function f. For example, in the two dimensional case the values x(n) of the nodes 222 of the pooling layer 212 can be calculated based on the values x(n-1) of the nodes 221 of the preceding layer 211 as

$$x^{(n)}[i,j] = f(x^{(n-1)}[id_1, jd_2], ..., x^{(n-1)}[id_1+d_1-1, jd_2+d_2-1]) .$$

In other words, by using a pooling layer 212 the number of nodes 221, 222 can be reduced, by replacing a number d1 d2 of neighbouring nodes 221 in the preceding layer 211 with a single node 222 being calculated as a function of the values of said number of neighbouring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced. This leads to the amount of computation in the network being reduced and to a control of overfitting.

In the embodiment of fig. 7, the pooling layer 212 is a max-pooling, replacing four neighbouring nodes with only one node, the value being the maximum of the values of the four neighbouring nodes. The maxpooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In the embodiment of fig. 7, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = max(0,x)$, the tangent hyperbolics function or the sigmoid function.

In particular, convolutional neural networks 200 can be trained based on the backpropagation algorithm. For preventing overfitting, methods of regularization can be used, e.g. dropout of nodes 220, ... , 224, stochastic pooling, use of artificial data, weight decay based on the L1 or the L2 norm, or max norm constraints.

When 2D CNN illustrated in fig. 7 is adapted to a 3D CNN, the 3D CNN may comprise an input layer, convolutional layers, pooling layers, fully connected layers, and an output layer. 3D CNN can include multiple instances of convolutional, pooling, and fully connected layers. The arrangement of these layers can vary. Fully connected layers may be arranged next to the output layer.

In a 3D CNN, the nodes within a layer may be arranged as a 3-dimensional matrix or image. For example, in the three-dimensional case, the value of a node indexed with i, j, and k in the nth layer can be denoted as x(n)[i,j,k]. The arrangement of nodes may not directly impact the calculations within the network, which may be determined by the structure and weights of the connections.

A convolutional layer may be characterized by the convolution operations it performs, using a set number of kernels. These operations may be extended to 3D, where a convolution may be defined for a 3D volume of input data. The k-th kernel may become a 3-dimensional matrix (e.g., 3x3x3), which may interact with the input volume to produce a convolved output. The number of nodes in a convolutional layer may become a function of the nodes in the preceding layer and the number of kernels, effectively adding or expanding the depth dimension in the node arrangement.

If the nodes of the preceding layer are arranged in a 3-dimensional matrix, using multiple kernels may add a depth dimension, making the convolutional layer's node arrangement a 4-dimensional matrix. The depth dimension's size may increase by a factor equivalent to the number of kernels used.

The use of convolutional layers may allow for the exploitation of spatially local correlations in the input data 5002, 5004 by enforcing local connectivity patterns between nodes of adjacent layers. Each node may be connected to a region of the nodes in the preceding layer, extending to three dimensions in 3D CNN.

For example, the input layer may comprise nodes arranged as a 3-dimensional matrix (e.g., 6x6x6). The convolutional layer following it may have its nodes arranged as multiple 3-dimensional matrices (e.g., 6x6x6 matrices for each kernel), or equivalently, as a 4-dimensional matrix to represent both spatial and depth dimensions.

Pooling layers in a 3D CNN may perform operations over 3D volumes, reducing the spatial dimensions and the depth dimension. For instance, a 3D max-pooling layer may reduce the size of each 3-dimensional volume of nodes by applying the pooling operation across spatial and depth dimensions.

Fully connected layers in 3D CNN may remain similar to fully connected layers of 2D CNN, but fully connected layers in 3D CNN may integrate inputs from a 3-dimensional or 4-dimensional arrangement of nodes, flattening them into a single vector for processing.

The output layer of 3D CNN, often employing a Softmax function for classification, may treat the values from the preceding fully connected layer to represent probabilities across different categories, with the sum of all output node values equating to 1.

Additionally, 3D CNNs can incorporate ReLU layers and alike, with each node's value calculated by applying an activation function to the corresponding node in the preceding layer. Training, including the prevention of overfitting, may follow similar principles but accounts for the 3-dimensional nature of the input data 5002, 5004 and the third dimension in 3D CNN as compared to 2D slice and 2D CNN.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustration, techniques have been disclosed that enable determining a 3D segmentation data based on 3D CT imaging data sets. Similar techniques the disclosed techniques may also be employed for determining 3D segmentation data based on other 3D imaging data sets, e.g., Magnetic Resonance Imaging (MRI) imaging data sets.

**EP 4 625 316 A1**

For further illustration, techniques have been disclosed for determining 3D segmentation data - e.g., segmentation maps, a circumference of an object of interest, a heat map, etc. - for segmenting a liver lesion. Other types of lesions or other types of regions of interest, e.g., certain organs etc. may be likewise segmented.

For still further illustration, it would also be possible to segment multiple objects. Here, it would be possible to provide segmentation data that includes a semantic information, the semantic information discriminating between multiple objects.

List of Reference

1000: a computer-implemented method for providing output data (using a trained function);
S102: receiving input data;
S103: pre-processing of input data;
S104: applying a trained function to the input data;
S106: generating output data;
S108: providing the output data;
S110: post processing of input data;
2000: a system for using a trained function;
2002: a first interface configured for receiving input data;
2004: a second interface configured for providing output data;
2006: a computation unit;
2008: a computer processor;
2010: computer readable instructions;
3000: a computer-implemented method for training a training function;
S302: receiving training input data;
S304: providing the training input data to a training function;
S306: embedding the training input data;
S308: receiving training output data;
S310: generating a trained function;
S312: providing the trained function for use;
4000: a system for training a training function;
4002: a first training computer interface;
4004: a second training computer interface;
4006: a third training computer interface;
4008: a computer processor;
4010: computer readable instructions;
4012: training computation unit;
5000: an encoder-decoder neural network architecture;
5002: input data comprising an image dataset taken at a reference phase;
5004: input data comprising image datasets taken at remaining phases;
5006: an encoder part;
5006a: a first encoder branch;
5006b: a second encoder branch;
5008: a decoder part;
5010: a spatial aggregation module, SA;
5012: output data comprising a 3D heatmap;
5014: output data comprising a 3D segmentation mask;
LE1, ..., LEm: layers of a neuronal network associated with the encoder part 5006;
LD1, ..., LDk: layers of a neuronal network associated with the decoder part 5008;
P1: a reference phase at time t1;
P2, ..., Pn: remaining phases at remaining times t2, ..., tn;
x, y, z: spatial coordinates;
F1, F2, F3: encoded feature information;
Agg_F4: spatially aggregated feature information;
100: a deep neuronal network;
110, ..., 113: layers of the deep neuronal network;
120, ..., 132: nodes of the deep neuronal network;

140, ..., 142: edges of the deep neuronal network;

200: a convolutional neural network;

210: an input layer of the convolutional neural network;

211: a convolutional layer of the convolutional neural network;

212: a pooling layer of the convolutional neural network;

213: a fully connected layer of the convolutional neural network;

214: an output layer of the convolutional neural network;

220, ..., 224: nodes of the convolutional neural network.

**Claims**

1. A computer-implemented method (1000) for providing output data, comprising:

   - receiving (S102), by a first computer interface, input data (5002, 5004),
   - applying (S104) a trained function (5000) to the input data (5002, 5004),
   - generating (S106), by the trained function (5000), output data (5012, 5014), wherein the output data is associated with the input data,
   - providing (S108), via a second computer interface, the output data (5012, 5014),

   wherein the input data (5002, 5004) comprises a multi-phase three-dimensional, 3D, computed tomography, CT, imaging dataset of a liver lesion associated with a patient, and

   wherein the output data (5012, 5014) comprises 3D segmentation data (5012; 5014) for locating the liver lesion in the input data (5002, 5004).

2. The computer-implemented method of claim 1, wherein the trained function (5000) comprises:

   - an encoder part (5006) for encoding each two-dimensional, 2D, slice of each phase (P1, P2, ..., Pn) from the input data (5002, 5004) into encoded feature information (F1, F2, F3),
   - a spatial aggregation, SA, module (5010) for aggregating the encoded feature information (F1, F2, F3) into spatially aggregated feature information (Agg_F4), and
   - a decoder part (5008) for decoding the spatially aggregated feature information (Agg_F4) into the 3D segmentation data (5012; 5014),

   wherein the encoder part (5006) comprises a first branch (5006a) receiving 2D slices of the input data (5002) associated with a reference phase (P1) and a second branch (5006b) receiving 2D slices of the input data (5004) associated with one or more remaining phases (P2, ..., Pn), each of the first branch (5006a) and the second branch (5006b) providing the encoded feature information (F1, F2, F3) to the SA module (5010) for aggregation, and

   wherein each of the first branch (5006a) and the second branch (5006b) of the encoder part (5006) comprises multiple stacked layers (LE1, ..., Lem; 110-113; 210-214) of a neuronal network (100; 200) providing the encoded feature information (F1, F2, F3) to the SA module (5010) for aggregation into the spatially aggregated feature information (Agg_F4).

3. The computer-implemented method of any one of the preceding claims, wherein the input data is pre-processed (S103) by a pre-processing algorithm, the pre-processing algorithm comprising:

   - manually selecting, for each phase from the multi-phase 3D CT imaging dataset, a 2D slice based on a diameter of a target lesion, indicating a center or a diameter of the target lesion and receiving (S102), via the first computer interface, the center or the diameter of the target lesion as a part of the input data (5002, 5004); or
   - automatically detecting, by an additional trained function, a bounding-box or coordinates of a centroid associated with the target lesion on a 2D slice for each phase from the input data (5002, 5004) based on the diameter of the target lesion, and receiving (S102), via the first computer interface, by the trained function, the bounding-box or the coordinates of the centroid as a part of the input data (5002, 5004),

   wherein the additional trained function comprises a deep neuronal network (100; 200) configured to receive the input data (5002, 5004) and provide the bounding box or the coordinates of the centroid to the trained function as a part of the input data (5002, 5004).

4. The computer-implemented method of claim 2, wherein the pre-processing (S103) further comprises:

- registering 2D slices of the input data (5002, 5004) associated with different phases (P1, ..., Pn) according to a mesh-based algorithm, or according to a point-cloud-based algorithm, wherein the mesh-based algorithm comprises providing a mesh having a plurality of interconnected data nodes representing a geometrical structure of a lesion within images of the input data (5002, 5004) for aligning or matching 2D slices associated with each phase (P1, ..., Pn) from the input data (5002, 5004) to the geometrical structure of the lesion,

wherein the point-cloud-based algorithm comprises providing a point cloud comprising a plurality of data points representing the geometrical structure of the lesion within the images of the input data (5002, 5004) for identifying a geometrical transformation that aligns corresponding points in the point cloud for spatial correspondence between the 2D slices of every phase (P1, ..., Pn) from the input data (5002, 5004).

5. The computer-implemented method of any of the preceding claims, wherein the output data is post-processed (S110) by a post-processing algorithm, the post-processing algorithm comprising:

- rendering, by a computer processor (2008), the 3D segmentation data (5015; 5014),
- calculating, by the computer processor (2008), one or more parameters associated with the target lesion from the 3D segmentation data (5012; 5014), wherein the one or more parameters comprise any one of a largest diameter, a volume, a lesion type, a lesion's contour, a minimum and/or a maximum intensity of each lesion; and
- providing the one or more parameters via the second computer interface (2004),

wherein the 3D segmentation data (5012, 5014) comprises a three dimensional, 3D, heat map and/or three dimensional segmentation mask obtainable by generating, by the trained function, the three-dimensional, 3D, heatmap and converting the generated 3D heatmap of the output data (5012) into a 3D binary mask by applying a predefined threshold to the generated 3D heatmap of the output data (5012) and identifying one or more connected components within the binary mask.

6. A computer system (2000), comprising:

- a first interface (2002), configured for receiving input data (5002, 5004),
- a second interface (2004), configured for providing output data (5012, 5014),
- a computation unit (2006) comprising a computer processor (2008) configured for applying a trained function (5000) trained by a machine learning algorithm to the input data (5002, 5004),
wherein the output data (5012, 5014) is generated by the trained function (5000), and
wherein the input data (5002, 5004) comprises a multi-phase three-dimensional, 3D, computed tomography, CT, imaging dataset of a liver lesion associated with a patient, and
wherein the output data (5012, 5014) comprises a 3D segmentation data (5012; 5014) for locating the liver lesion in the input data (5002, 5004).

7. The computer system of claim 6, wherein the trained function (5000) comprises:

- an encoder part (5006) for encoding each 2D slice of each phase (P1, P2, ..., Pn) from the input data (5002, 5004) that is a multi-phase 3D CT imaging dataset into encoded feature information (F1, F2, F3),
- a spatial aggregation, SA, module (5010) for aggregating the encoded feature information (F1, F2, F3) into spatially aggregated feature information (Agg_F4), and
- a decoder part (5008) for decoding the spatially aggregated feature information (Agg_F4) into the output data (5012, 5014) comprising 3D segmentation data (5012; 5014),
wherein the encoder part (5006) comprises a first branch (5006a) receiving 2D slices of the input data (5002) associated with a reference phase (P1) and a second branch (5006b) receiving 2D slices of the input data (5004) associated with one or more remaining phases (P2, ..., Pn), each of the first branch (5006a) and the second branch (5006b) providing the encoded feature information (F1, F2, F3) to the SA module (5010) for aggregation, and
wherein each of the first branch (5006a) and the second branch (5006b) of the encoder part (5006) comprises multiple stacked layers (LE1, ..., Lem; 110-113; 210-214) of a neuronal network (100; 200) providing the encoded feature information (F1, F2, F3) to the SA module (5010) for aggregation into the spatially aggregated feature information (Agg_F4).

8. The computer system of claim 6 or 7, wherein the computation unit (2006) is further configured to pre-process the input data (5002, 5004) according to a pre-processing algorithm, the pre-processing algorithm comprising:

- manually selecting, for each phase from the multi-phase 3D CT imaging dataset, a 2D slice, wherein a target lesion has the largest diameter, indicating a center or a diameter of the target lesion and receiving (S102), via the first computer interface, the center or the diameter of the target lesion as a part of the input data (5002, 5004); or
- automatically detecting, by an additional trained function, a bounding-box or coordinates of a centroid associated with the target lesion on a 2D slice for each phase from the input data (5002, 5004) wherein the target lesion has the largest diameter, and receiving (S102), via the first computer interface, by the trained function, the bounding-box or the coordinates of the centroid as a part of the input data (5002, 5004),

wherein the additional trained function comprises a deep neuronal network (100; 200) configured to receive the input data (5002, 5004) and provide the bounding box or the coordinates of the centroid to the trained function as a part of the input data (5002, 5004).

9. The computer system of claim 8, wherein the pre-processing algorithm further comprises:

- registering 2D slices of the input data (5002, 5004) associated with different phases (P1, ..., Pn) according to a mesh-based algorithm, or according to a point-cloud-based algorithm, wherein the mesh-based algorithm comprises providing a mesh having a plurality of interconnected data nodes representing a geometrical structure of a lesion within images of the input data (5002, 5004) for aligning or matching two-dimensional, 2D, slices associated with each phase (P1, ..., Pn) from the multi-phase 3D CT imaging dataset (the input data 5002, 5004) to the geometrical structure of the lesion,

wherein the point-cloud-based algorithm comprises providing a point cloud comprising a plurality of data points representing the geometrical structure of the lesion within the images of the input data (5002, 5004) for identifying a geometrical transformation that aligns corresponding points in the point cloud for spatial correspondence between the 2D slices of every phase (P1, ..., Pn) from the input data (5002, 5004).

10. The computer system of any of claims 6-9, wherein the computation unit (2006) is configured to post-process the output data (5012, 5014) according to a post-processing algorithm, the post-processing algorithm comprising:

- rendering, by a computer processor (2008), the output data (5012, 5014) comprising 3D segmentation data (5015; 5014),
- calculating, by the computer processor (2008), one or more parameters associated with the target lesion from the 3D segmentation data (5012; 5014), wherein the one or more parameters comprise any one of a largest diameter, a volume, a lesion type, a lesion's contour, a minimum and/or a maximum intensity of each lesion; and
- providing the one or more parameters via the second computer interface (2004),

wherein the 3D segmentation data (5012, 5014) comprises a three dimensional, 3D, heat map and/or three dimensional segmentation mask obtainable by generating, by the trained function, the three-dimensional, 3D, heatmap and converting the generated 3D heatmap of the output data (5012) into a 3D binary mask by applying a predefined threshold to the generated 3D heatmap of the output data (5012) and identifying one or more connected components within the binary mask.

11. A computer implemented method (3000) for providing a trained function (5000), the method comprising:

- receiving (S302), by a first training computer interface (4002), training input data (5002, 5004),
- providing (S304), by a computer processor (4008), the training input data to a training function (5000),
- embedding (S306), by the training function (5000), the training input data (5002, 5004),
- receiving (S308), via a second training computer interface (4004), training output data (5012, 5014), wherein the training output data is associated with the training input data,
- generating (S310), by the computer processor (4008), a trained function comprising embedded training input data,
- providing (S312), via a third training computer interface (4006), the trained function (5000) for use,

wherein the training input data (5002, 5004) comprises one or more multi-phase three-dimensional, 3D, computed tomography, CT, imaging datasets of one or more liver lesions, wherein each imaging dataset is associated with a patient.

12. The computer-implemented method of claim 11,

wherein the trained function (5000) comprises:

- an encoder part (5006) for encoding each two-dimensional, 2D, slice of each phase (P1, P2, ..., Pn) from the input data (5002, 5004) into encoded feature information (F1, F2, F3),
- a spatial aggregation, SA, module (5010) for aggregating the encoded feature information (F1, F2, F3) into spatially aggregated feature information (Agg_F4), and
- a decoder part (5008) for decoding the spatially aggregated feature information (Agg_F4) into the 3D segmentation data (5012; 5014),

wherein the encoder part (5006) comprises a first branch (5006a) receiving 2D slices of the imaging datasets of the input data (5002) associated with a reference phase (P1) and a second branch (5006b) receiving 2D slices of the imaging datasets of the input data (5004) associated with one or more remaining phases (P2, ..., Pn), each of the first branch (5006a) and the second branch (5006b) providing the encoded feature information (F1, F2, F3) to the SA module (5010) for aggregation, and

wherein each of the first branch (5006a) and the second branch (5006b) of the encoder part (5006) comprises multiple stacked layers (LE1, ..., Lem; 110-113; 210-214) of a neuronal network (100; 200) providing the encoded feature information (F1, F2, F3) to the SA module (5010) for aggregation into the spatially aggregated feature information (Agg_F4).

**13.** The computer-implemented method of claim 11 or 12, further comprising:

- detecting one or more regions of interest, ROIs, for each lesion within each image of the input data (5002, 5004), wherein the detecting comprises determining a centre of mass or centroid of each lesion;
- cropping each image of the input data (5002, 5004) around the centre of mass or centroid;
- encoding cropped images by the encoder part (5006);
- matching the centres of mass or centroids across the multi-phase 3D CT imaging dataset to establish a representation of the lesion's position in three-dimensional, 3D, space;
- aggregating, by the SA module (5010), encoded feature information (F1, F2, F3) into spatially aggregated feature information (Agg_F4);
- generating, by the decoder part (5008) decoding the spatially aggregated feature information (Agg_F4), a three-dimensional, 3D, heat map as a part of the output data (5012), wherein the 3D heat map is a 3D graphical representation indicating spatial location of each lesion across the multi-phase 3D CT imaging dataset; and
- generating, by applying a threshold to the generated 3D heatmap, a 3D segmentation mask as a part of the output data (5014).

**14.** The computer-implemented method of any one of claims 11-13, further comprising:

- training an additional function comprising a deep neuronal network (100; 200) to provide an additional trained function, the additional function being trained by embedding additional training data associated with a plurality of bounding-boxes or coordinates of a plurality of centroids each associated with a lesion present in the training input data (5002, 5004) provided to the additional function together with the plurality of bounding-boxes or coordinates of the plurality of centroids via the first training computer interface (4002);
wherein the additional trained function is trained to generate output data being a bounding-box or coordinates of a centroid associated with a target lesion; and
- chaining the additional trained function with the trained function of any one of claims 1-11 such that the output data generated by the additional trained function is provided as an additional input data in addition to the input data (5002, 5004) provided to the trained function of any one of claims 1-11 for automatic detection of a bounding-box or coordinates of a centroid associated with a target lesion present in the input data (5002, 5004).

**15.** The computer-implemented method of any one of claims 11-14, further comprising:

- receiving, by the first training computer interface, a three-dimensional, 3D, ground truth segmentation mask corresponding to the 3D heatmap;
- calculating a weighted loss function by integrating a cross-entropy loss component and a dice loss component,

wherein the cross-entropy loss component quantifies dissimilarity between predicted and actual probability distributions of class labels, wherein the actual probability distribution is a binary vector representing the true class, where the probability is one (1) for the correct class representing a lesion and zero (0) for other classes that do not represent a lesion, and
wherein the dice loss component quantifies the spatial overlap between predicted and the ground truth segmentation masks;

- iteratively updating one or more hyperparameters of the trained function based on the calculated weighted loss function, wherein the one or more hyperparameters comprise any one of or a combination thereof a type of optimizer, a learning rate and/or a regularization weight, and
- generating the trained function comprising the one or more hyperparameters.

**16.** The computer-implemented method of any one of claims 11-15, further comprising evaluating the trained function according to an evaluation algorithm, the evaluation algorithm comprises:

- receiving, via the first training computer interface, the training input data by the trained function of any one of claims 11-15;
- generating, by the trained function, a 3D heatmap;
- generating a binary mask from the generated 3D heatmap by applying a predetermined threshold to the 3D heatmap;
- identifying a 3D segmentation mask that aligns with the center of the lesion based on identifying connected components within the binary mask by: identifying spatial proximity between pixels of the binary mask, assigning one or more labels to the one or more connected components for distinguishing the one or more components, generating an output representation comprising the labelled connected components,
- generating the identified 3D segmentation mask and providing the generated 3D segmentation mask via the second training computer interface (4004) as training output data (5014);
- providing, via the first training computer interface (4002), a comparative ground truth segmentation mask associated with the training input data,
- comparing the generated 3D segmentation mask with the comparative ground truth segmentation mask; wherein the comparative ground truth segmentation mask is an annotated set of binary images that serves as a reference delineating the true boundaries or labels of the connected components;
- calculating an evaluation metric by comparing the generated 3D segmentation mask and the comparative ground truth segmentation mask, the evaluation metric comprising one or more performance characteristics of the trained function and providing the calculated evaluation metric via the second (4004) or the third training computer interface (4006).

**17.** A computer program product comprising computer readable instructions (2010; 4010) which, when executed by a computer processor, cause the computer processor to carry out the method of one of claims 1 to 5 and/or any one of claims 11-16.

**18.** A training system (4000), comprising:

- a first training computer interface (4002) configured for receiving training input data (5002, 5004),
- a training computation unit (4012) comprising a computer processor (4008) configured for training a training function (5000) by providing the training input data (5002, 5004) to the training function (5000) for embedding, by the training function, the training input data, and the computer processor (4008) further configured for generating the trained function (5000) comprising the embedded training data,
- a second training computer interface (4004) configured for providing output data (5012, 5014), wherein the output data is associated with the input data, and
- a third training computer interface (4006) configured for providing the trained function (5000) for use,

wherein the training input data (5002, 5004) comprises one or more multi-phase three-dimensional, 3D, computed tomography, CT, imaging datasets of one or more liver lesions, wherein each imaging dataset is associated with a patient.

**Amended claims in accordance with Rule 137(2) EPC.**

**1.** A computer-implemented method (1000) for providing output data, comprising:

- receiving (S102), by a first computer interface, input data (5002, 5004),
- applying (S104) a trained function (5000) to the input data (5002, 5004),

- generating (S106), by the trained function (5000), output data (5012, 5014), wherein the output data is associated with the input data,
- providing (S108), via a second computer interface, the output data (5012, 5014),

wherein the input data (5002, 5004) comprises a multi-phase three-dimensional, 3D, computed tomography, CT, imaging dataset of a liver lesion associated with a patient,

wherein the output data (5012, 5014) comprises 3D segmentation data (5012; 5014) for locating the liver lesion in the input data (5002, 5004), and

wherein the output data is post-processed (S110) by a post-processing algorithm, the post-processing algorithm comprising:

- rendering, by a computer processor (2008), the 3D segmentation data (5015; 5014),
- calculating, by the computer processor (2008), one or more parameters associated with the target lesion from the 3D segmentation data (5012; 5014), wherein the one or more parameters comprise any one of a largest diameter, a volume, a lesion type, a lesion's contour, a minimum and/or a maximum intensity of each lesion; and
- providing the one or more parameters via the second computer interface (2004),

wherein the 3D segmentation data (5012, 5014) comprises a three dimensional, 3D, heat map and/or three dimensional segmentation mask obtainable by generating, by the trained function, the three-dimensional, 3D, heatmap and converting the generated 3D heatmap of the output data (5012) into a 3D binary mask by applying a predefined threshold to the generated 3D heatmap of the output data (5012) and identifying one or more connected components within the binary mask.

2. The computer-implemented method of claim 1, wherein the trained function (5000) comprises:

- an encoder part (5006) for encoding each two-dimensional, 2D, slice of each phase (P1, P2, ..., Pn) from the input data (5002, 5004) into encoded feature information (F1, F2, F3),
- a spatial aggregation, SA, module (5010) for aggregating the encoded feature information (F1, F2, F3) into spatially aggregated feature information (Agg_F4), and
- a decoder part (5008) for decoding the spatially aggregated feature information (Agg_F4) into the 3D segmentation data (5012; 5014),

wherein the encoder part (5006) comprises a first branch (5006a) receiving 2D slices of the input data (5002) associated with a reference phase (P1) and a second branch (5006b) receiving 2D slices of the input data (5004) associated with one or more remaining phases (P2, ..., Pn), each of the first branch (5006a) and the second branch (5006b) providing the encoded feature information (F1, F2, F3) to the SA module (5010) for aggregation, and

wherein each of the first branch (5006a) and the second branch (5006b) of the encoder part (5006) comprises multiple stacked layers (LE1, ..., Lem; 110-113; 210-214) of a neuronal network (100; 200) providing the encoded feature information (F1, F2, F3) to the SA module (5010) for aggregation into the spatially aggregated feature information (Agg_F4).

3. The computer-implemented method of any one of the preceding claims, wherein the input data is pre-processed (S103) by a pre-processing algorithm, the pre-processing algorithm comprising:

- manually selecting, for each phase from the multi-phase 3D CT imaging dataset, a 2D slice based on a diameter of a target lesion, indicating a center or a diameter of the target lesion and receiving (S102), via the first computer interface, the center or the diameter of the target lesion as a part of the input data (5002, 5004); or
- automatically detecting, by an additional trained function, a bounding-box or coordinates of a centroid associated with the target lesion on a 2D slice for each phase from the input data (5002, 5004) based on the diameter of the target lesion, and receiving (S102), via the first computer interface, by the trained function, the bounding-box or the coordinates of the centroid as a part of the input data (5002, 5004),

wherein the additional trained function comprises a deep neuronal network (100; 200) configured to receive the input data (5002, 5004) and provide the bounding box or the coordinates of the centroid to the trained function as a part of the input data (5002, 5004).

4. The computer-implemented method of claim 2, wherein the pre-processing (S103) further comprises:

- registering 2D slices of the input data (5002, 5004) associated with different phases (P1, ..., Pn) according to a mesh-based algorithm, or according to a point-cloud-based algorithm, wherein the mesh-based algorithm comprises providing a mesh having a plurality of interconnected data nodes representing a geometrical structure of a lesion within images of the input data (5002, 5004) for aligning or matching 2D slices associated with each phase (P1, ..., Pn) from the input data (5002, 5004) to the geometrical structure of the lesion, wherein the point-cloud-based algorithm comprises providing a point cloud comprising a plurality of data points representing the geometrical structure of the lesion within the images of the input data (5002, 5004) for identifying a geometrical transformation that aligns corresponding points in the point cloud for spatial correspondence between the 2D slices of every phase (P1, ..., Pn) from the input data (5002, 5004).

5. A computer system (2000), comprising:

- a first interface (2002), configured for receiving input data (5002, 5004),
- a second interface (2004), configured for providing output data (5012, 5014),
- a computation unit (2006) comprising a computer processor (2008) configured for applying a trained function (5000) trained by a machine learning algorithm to the input data (5002, 5004), wherein the output data (5012, 5014) is generated by the trained function (5000), and

wherein the input data (5002, 5004) comprises a multi-phase three-dimensional, 3D, computed tomography, CT, imaging dataset of a liver lesion associated with a patient, wherein the output data (5012, 5014) comprises a 3D segmentation data (5012; 5014) for locating the liver lesion in the input data (5002, 5004), and wherein the computation unit (2006) is configured to post-process the output data (5012, 5014) according to a post-processing algorithm, the post-processing algorithm comprising:

- rendering, by a computer processor (2008), the output data (5012, 5014) comprising 3D segmentation data (5015; 5014),
- calculating, by the computer processor (2008), one or more parameters associated with the target lesion from the 3D segmentation data (5012; 5014), wherein the one or more parameters comprise any one of a largest diameter, a volume, a lesion type, a lesion's contour, a minimum and/or a maximum intensity of each lesion; and
- providing the one or more parameters via the second computer interface (2004),

wherein the 3D segmentation data (5012, 5014) comprises a three dimensional, 3D, heat map and/or three dimensional segmentation mask obtainable by generating, by the trained function, the three-dimensional, 3D, heatmap and converting the generated 3D heatmap of the output data (5012) into a 3D binary mask by applying a predefined threshold to the generated 3D heatmap of the output data (5012) and identifying one or more connected components within the binary mask.

6. The computer system of claim 5, wherein the trained function (5000) comprises:

- an encoder part (5006) for encoding each 2D slice of each phase (P1, P2, ..., Pn) from the input data (5002, 5004) that is a multi-phase 3D CT imaging dataset into encoded feature information (F1, F2, F3),
- a spatial aggregation, SA, module (5010) for aggregating the encoded feature information (F1, F2, F3) into spatially aggregated feature information (Agg_F4), and
- a decoder part (5008) for decoding the spatially aggregated feature information (Agg_F4) into the output data (5012, 5014) comprising 3D segmentation data (5012; 5014), wherein the encoder part (5006) comprises a first branch (5006a) receiving 2D slices of the input data (5002) associated with a reference phase (P1) and a second branch (5006b) receiving 2D slices of the input data (5004) associated with one or more remaining phases (P2, ..., Pn), each of the first branch (5006a) and the second branch (5006b) providing the encoded feature information (F1, F2, F3) to the SA module (5010) for aggregation, and wherein each of the first branch (5006a) and the second branch (5006b) of the encoder part (5006) comprises multiple stacked layers (LE1, ..., Lem; 110-113; 210-214) of a neuronal network (100; 200) providing the encoded feature information (F1, F2, F3) to the SA module (5010) for aggregation into the spatially aggregated feature information (Agg_F4).

7. The computer system of claim 5 or 6, wherein the computation unit (2006) is further configured to pre-process the input data (5002, 5004) according to a pre-processing algorithm, the pre-processing algorithm comprising:

- manually selecting, for each phase from the multi-phase 3D CT imaging dataset, a 2D slice, wherein a target lesion has the largest diameter, indicating a center or a diameter of the target lesion and receiving (S102), via the first computer interface, the center or the diameter of the target lesion as a part of the input data (5002, 5004); or
- automatically detecting, by an additional trained function, a bounding-box or coordinates of a centroid associated with the target lesion on a 2D slice for each phase from the input data (5002, 5004) wherein the target lesion has the largest diameter, and receiving (S102), via the first computer interface, by the trained function, the bounding-box or the coordinates of the centroid as a part of the input data (5002, 5004),
wherein the additional trained function comprises a deep neuronal network (100; 200) configured to receive the input data (5002, 5004) and provide the bounding box or the coordinates of the centroid to the trained function as a part of the input data (5002, 5004).

8. The computer system of claim 7, wherein the pre-processing algorithm further comprises:

- registering 2D slices of the input data (5002, 5004) associated with different phases (P1, ..., Pn) according to a mesh-based algorithm, or according to a point-cloud-based algorithm, wherein the mesh-based algorithm comprises providing a mesh having a plurality of interconnected data nodes representing a geometrical structure of a lesion within images of the input data (5002, 5004) for aligning or matching two-dimensional, 2D, slices associated with each phase (P1, ..., Pn) from the multi-phase 3D CT imaging dataset (the input data 5002, 5004) to the geometrical structure of the lesion,
wherein the point-cloud-based algorithm comprises providing a point cloud comprising a plurality of data points representing the geometrical structure of the lesion within the images of the input data (5002, 5004) for identifying a geometrical transformation that aligns corresponding points in the point cloud for spatial correspondence between the 2D slices of every phase (P1, ..., Pn) from the input data (5002, 5004).

9. A computer implemented method (3000) for providing a trained function (5000), the method comprising:

- receiving (S302), by a first training computer interface (4002), training input data (5002, 5004),
- providing (S304), by a computer processor (4008), the training input data to a training function (5000),
- embedding (S306), by the training function (5000), the training input data (5002, 5004),
- receiving (S308), via a second training computer interface (4004), training output data (5012, 5014), wherein the training output data is associated with the training input data,
- generating (S310), by the computer processor (4008), a trained function comprising embedded training input data,
- providing (S312), via a third training computer interface (4006), the trained function (5000) for use,
wherein the training input data (5002, 5004) comprises one or more multi-phase three-dimensional, 3D, computed tomography, CT, imaging datasets of one or more liver lesions, wherein each imaging dataset is associated with a patient.

10. The computer-implemented method of claim 9,

wherein the trained function (5000) comprises:

- an encoder part (5006) for encoding each two-dimensional, 2D, slice of each phase (P1, P2, ..., Pn) from the input data (5002, 5004) into encoded feature information (F1, F2, F3),
- a spatial aggregation, SA, module (5010) for aggregating the encoded feature information (F1, F2, F3) into spatially aggregated feature information (Agg_F4), and
- a decoder part (5008) for decoding the spatially aggregated feature information (Agg_F4) into the 3D segmentation data (5012; 5014),

wherein the encoder part (5006) comprises a first branch (5006a) receiving 2D slices of the imaging datasets of the input data (5002) associated with a reference phase (P1) and a second branch (5006b) receiving 2D slices of the imaging datasets of the input data (5004) associated with one or more remaining phases (P2, ..., Pn), each of the first branch (5006a) and the second branch (5006b) providing the encoded feature information (F1, F2, F3) to the SA module (5010) for aggregation, and
wherein each of the first branch (5006a) and the second branch (5006b) of the encoder part (5006) comprises multiple stacked layers (LE1, ..., Lem; 110-113; 210-214) of a neuronal network (100; 200) providing the encoded feature information (F1, F2, F3) to the SA module (5010) for aggregation into the spatially aggregated feature information (Agg_F4).

11. The computer-implemented method of claim 9 or 10, further comprising:

- detecting one or more regions of interest, ROIs, for each lesion within each image of the input data (5002, 5004), wherein the detecting comprises determining a centre of mass or centroid of each lesion;
- cropping each image of the input data (5002, 5004) around the centre of mass or centroid;
- encoding cropped images by the encoder part (5006);
- matching the centres of mass or centroids across the multi-phase 3D CT imaging dataset to establish a representation of the lesion's position in three-dimensional, 3D, space;
- aggregating, by the SA module (5010), encoded feature information (F1, F2, F3) into spatially aggregated feature information (Agg_F4);
- generating, by the decoder part (5008) decoding the spatially aggregated feature information (Agg_F4), a three-dimensional, 3D, heat map as a part of the output data (5012), wherein the 3D heat map is a 3D graphical representation indicating spatial location of each lesion across the multi-phase 3D CT imaging dataset; and
- generating, by applying a threshold to the generated 3D heatmap, a 3D segmentation mask as a part of the output data (5014).

12. The computer-implemented method of any one of claims 9-11, further comprising:

- training an additional function comprising a deep neuronal network (100; 200) to provide an additional trained function, the additional function being trained by embedding additional training data associated with a plurality of bounding-boxes or coordinates of a plurality of centroids each associated with a lesion present in the training input data (5002, 5004) provided to the additional function together with the plurality of bounding-boxes or coordinates of the plurality of centroids via the first training computer interface (4002);
wherein the additional trained function is trained to generate output data being a bounding-box or coordinates of a centroid associated with a target lesion; and
- chaining the additional trained function with the trained function of any one of claims 1-11 such that the output data generated by the additional trained function is provided as an additional input data in addition to the input data (5002, 5004) provided to the trained function of any one of claims 1-11 for automatic detection of a bounding-box or coordinates of a centroid associated with a target lesion present in the input data (5002, 5004).

13. The computer-implemented method of any one of claims 9-12, further comprising:

- receiving, by the first training computer interface, a three-dimensional, 3D, ground truth segmentation mask corresponding to the 3D heatmap;
- calculating a weighted loss function by integrating a cross-entropy loss component and a dice loss component,

wherein the cross-entropy loss component quantifies dissimilarity between predicted and actual probability distributions of class labels, wherein the actual probability distribution is a binary vector representing the true class, where the probability is one (1) for the correct class representing a lesion and zero (0) for other classes that do not represent a lesion, and
wherein the dice loss component quantifies the spatial overlap between predicted and the ground truth segmentation masks;

- iteratively updating one or more hyperparameters of the trained function based on the calculated weighted loss function, wherein the one or more hyperparameters comprise any one of or a combination thereof a type of optimizer, a learning rate and/or a regularization weight, and
- generating the trained function comprising the one or more hyperparameters.

14. The computer-implemented method of any one of claims 9-13, further comprising evaluating the trained function according to an evaluation algorithm, the evaluation algorithm comprises:

- receiving, via the first training computer interface, the training input data by the trained function of any one of claims 9-13;
- generating, by the trained function, a 3D heatmap;
- generating a binary mask from the generated 3D heatmap by applying a predetermined threshold to the 3D heatmap;
- identifying a 3D segmentation mask that aligns with the center of the lesion based on identifying connected components within the binary mask by: identifying spatial proximity between pixels of the binary mask, assigning one or more labels to the one or more connected components for distinguishing the one or more components, generating an output representation comprising the labelled connected components,
- generating the identified 3D segmentation mask and providing the generated 3D segmentation mask via the second training computer interface (4004) as training output data (5014);
- providing, via the first training computer interface (4002), a comparative ground truth segmentation mask associated with the training input data,
- comparing the generated 3D segmentation mask with the comparative ground truth segmentation mask; wherein the comparative ground truth segmentation mask is an annotated set of binary images that serves as a reference delineating the true boundaries or labels of the connected components;
- calculating an evaluation metric by comparing the generated 3D segmentation mask and the comparative ground truth segmentation mask, the evaluation metric comprising one or more performance characteristics of the trained function and providing the calculated evaluation metric via the second (4004) or the third training computer interface (4006).

15. A computer program product comprising computer readable instructions (2010; 4010) which, when executed by a computer processor, cause the computer processor to carry out the method of one of claims 1 to 4 and/or any one of claims 9-14.

16. A training system (4000), comprising:

- a first training computer interface (4002) configured for receiving training input data (5002, 5004),
- a training computation unit (4012) comprising a computer processor (4008) configured for training a training function (5000) by providing the training input data (5002, 5004) to the training function (5000) for embedding, by the training function, the training input data, and the computer processor (4008) further configured for generating the trained function (5000) comprising the embedded training data,
- a second training computer interface (4004) configured for providing output data (5012, 5014), wherein the output data is associated with the input data, and
- a third training computer interface (4006) configured for providing the trained function (5000) for use, wherein the training input data (5002, 5004) comprises one or more multi-phase three-dimensional, 3D, computed tomography, CT, imaging datasets of one or more liver lesions, wherein each imaging dataset is associated with a patient.

## FIG 1

## FIG 2

## FIG 3

3000

S302

S304

S306

S308

S310

S312

## FIG 4

4000

5002, 5004

4002

5012, 5014

4004

4006

5000

4012

4008

5000

4010

# FIG 5

# FIG 6

# FIG 7

Europäisches
Patentamt
European
Patent Office
Office européen
des brevets

# EUROPEAN SEARCH REPORT

**Application Number**

EP 24 46 5514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG YUE ET AL: "Multi-phase Liver Tumor Segmentation with Spatial Aggregation and Uncertain Region Inpainting", 21 September 2021 (2021-09-21), 20210921, PAGE(S) 68 - 77, XP047611026, [retrieved on 2021-09-21] | 1,2,5-7, 10-12, 15-18 | INV. G06T7/00 |
| Y | * Figure 1; Sections 2 and 3 * | 3,4,8,9, 13,14 | |
| Y | CN 116 912 301 A (UNIV ZHEJIANG; HANGZHOU PUJIAN MEDICAL TECH CO LTD) 20 October 2023 (2023-10-20) * Claims 1, 3 and 8 and corresponding passages * | 3,4,8,9, 13 | |
| Y | US 2012/070055 A1 (LIU DAVID [US] ET AL) 22 March 2012 (2012-03-22) * Paragraphs 0029 to 0046, Claims 1 and 11 * | 3,8,13 | |
| Y | CAI JINZHENG ET AL: "Deep Volumetric Universal Lesion Detection Using Light-Weight Pseudo 3D Convolution and Surface Point Regression", 29 September 2020 (2020-09-29), 20200929, PAGE(S) 3 - 13, XP047563976, [retrieved on 2020-09-29] * Figure 1 and corresponding passages * | 14 | **TECHNICAL FIELDS SEARCHED (IPC)** G06T G06N A61B |

The present search report has been drawn up for all claims

| Place of search | Date of completion of the search | Examiner |
|---|---|---|
| The Hague | 20 August 2024 | Thean, Andrew |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 46 5514

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2024

| Patent document cited in search report | | Publication date | Patent family member(s) | | Publication date |
|---|---|---|---|---|---|
| CN 116912301 | A | 20-10-2023 | NONE | | |
| US 2012070055 | A1 | 22-03-2012 | US | 2012070055 A1 | 22-03-2012 |
| | | | WO | 2012040410 A2 | 29-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- U-net: Convolutional networks for biomedical image segmentation. **RONNEBERGER, O.** ; **FISCHER, P.** ; **BROX, T.** Medical Image Computing and Computer-Assisted Intervention-MICCAI 2015: 18th International Conference, Munich, Germany, October 5-9, 2015, Proceedings. Springer International Publishing, 2015, vol. 18, 234-241

- **OUHMICH, FARID et al.** Liver tissue segmentation in multiphase CT scans using cascaded convolutional neural networks. *International journal of computer assisted radiology and surgery*, 2019, vol. 14, 1275-1284

- **XU, YINGYING et al.** PA-ResSeg: A phase attention residual network for liver tumor segmentation from multiphase CT images. *Medical Physics*, 2021, vol. 48 (7), 3752-3766

- Multi-phase liver tumor segmentation with spatial aggregation and uncertain region inpainting. **ZHANG, YUE et al.** Medical Image Computing and Computer Assisted Intervention-MICCAI 2021: 24th International Conference, Strasbourg, France, September 27-October 1, 2021, Proceedings. Springer International Publishing, 2021, 24